# EUROPEAN PATENT SPECIFICATION

(11) **EP 3 030 262 B1**
(45) Date of publication and mention of the grant of the patent: **09.10.2019**
(21) Application number: 14761281.6
(22) Date of filing: 08.08.2014
(51) Int. Cl.: A61K 39/395, C07K 14/54, C07K 14/705, C07K 16/28, A61P 35/00, C07K 14/715

(54) **COMBINED PHARMACEUTICAL COMPOSITION**
KOMBINIERTE PHARMAZEUTISCHE ZUSAMMENSETZUNG
COMPOSITION PHARMACEUTIQUE COMBINÉE

(30) Priority: 08.08.2013 EP 13003964
(43) Date of publication of application: 15.06.2016
(73) Proprietor: Cytune Pharma, 44100 Nantes (FR); Institut Gustave Roussy (IGR), 94805 Villejuif Cedex (FR)
(72) Inventor: BECHARD, David, 44360 Saint-Etienne de Montlu (FR); CHAPUT, Nathalie, 75015 Paris (FR); DESBOIS, Mélanie, CA-94117 San Francisco (US)
(74) Representative: Maiwald Patent- und Rechtsanwaltsgesellschaft mbH
(86) International application number: PCT/EP2014/002182
(87) International publication number: WO 2015/018529

(56) References cited:
- WO-A1-2012/175222
- WO-A2-2009/002562
- P. YU ET AL: "Simultaneous Blockade of Multiple Immune System Inhibitory Checkpoints Enhances Antitumor Activity Mediated by Interleukin-15 in a Murine Metastatic Colon Carcinoma Model", CLINICAL CANCER RESEARCH, vol. 16, no. 24, 5 October 2010 (2010-10-05), pages 6019-6028, XP055123618, ISSN: 1078-0432, DOI: 10.1158/1078-0432.CCR-10-1966
- MARIE VINCENT ET AL: "Tumor targeting of the IL-15 superagonist RLI by an anti-GD2 antibody strongly enhances its antitumor potency", INTERNATIONAL JOURNAL OF CANCER, vol. 133, no. 3, 1 August 2013 (2013-08-01), pages 757-765, XP055151554, ISSN: 0020-7136, DOI: 10.1002/ijc.28059
- MARIE VINCENT ET AL: "CS14-6. Development of two IL15 immunocytokines targeting either GD2- or CD20- tumoral bearing cells", CYTOKINE, ACADEMIC PRESS LTD, PHILADELPHIA, PA, US, vol. 56, no. 1, 1 October 2011 (2011-10-01), page 102, XP002664482, ISSN: 1043-4666, DOI: 10.1016/J.CYTO.2011.07.396 [retrieved on 2011-09-08]
- W. XU ET AL: "Efficacy and Mechanism-of-Action of a Novel Superagonist Interleukin-15: Interleukin-15 Receptor Su/Fc Fusion Complex in Syngeneic Murine Models of Multiple Myeloma", CANCER RESEARCH, vol. 73, no. 10, 3 May 2013 (2013-05-03), pages 3075-3086, XP055151674, ISSN: 0008-5472, DOI: 10.1158/0008-5472.CAN-12-2357
- JUNZO HAMANISHI ET AL: "PD-1/PD-L1 blockade in cancer treatment: perspectives and issues", INTERNATIONAL JOURNAL OF CLINICAL ONCOLOGY, vol. 21, no. 3, 22 February 2016 (2016-02-22), pages 462-473, XP055535295, GB ISSN: 1341-9625, DOI: 10.1007/s10147-016-0959-z
- DATABASE EMBASE [Online] ELSEVIER SCIENCE PUBLISHERS, AMSTERDAM, NL; 20 July 2016 (2016-07-20), MITTICA G ET AL: "Immune checkpoint inhibitors: A new opportunity in the treatment of ovarian cancer?", Database accession no. EMB-20160548250 & MITTICA G ET AL: "Immune checkpoint inhibitors: A new opportunity in the treatment of ovarian cancer?", INTERNATIONAL JOURNAL OF MOLECULAR SCIENCES 20160720 MDPI AG CHE, vol. 17, no. 7, 20 July 2016 (2016-07-20), ISSN: 1661-6596

### SEQUENCE LISTING

<110> IGR
   CYTUNE
   BECHARD, David
   DESBOIS, Mélanie
   CHAPUT, Nathalie
<120> COMBINED PHARMACEUTICAL COMPOSITION
<130> CYT-B-0007-PCT1
<150> EP 13003964.7
   <151> 2013-08-08
<160> 17
<170> PatentIn version 3.5
<210> 1
   <211> 114
   <212> PRT
   <213> artificial sequence
<220>
   <223> mammalian interleukin 15 consensus sequence
<220>
   <221> MISC_FEATURE
   <222> (1)..(1)
   <223> X= N, S, T or I
<220>
   <221> MISC_FEATURE
   <222> (3)..(3)
   <223> X= V, H, I, Q or E
<220>
   <221> MISC_FEATURE
   <222> (4)..(4)
   <223> X= N, Y, F or D
<220>
   <221> MISC_FEATURE
   <222> (6)..(6)
   <223> X= I or R
<220>
   <221> MISC_FEATURE
   <222> (7)..(7)
   <223> X= S, N, L, Y, K or R
<220>
   <221> MISC_FEATURE
   <222> (10)..(10)
   <223> X= K, E, R or Q
<220>
   <221> MISC_FEATURE
   <222> (11)..(11)
   <223> X= K, T or R
<220>
   <221> MISC_FEATURE
   <222> (13)..(13)
   <223> X= E, D or Q
<220>
   <221> MISC_FEATURE
   <222> (14)..(14)
   <223> X= D, H, S or N
<220>
   <221> MISC_FEATURE
   <222> (16)..(16)
   <223> X= I or T
<220>
   <221> MISC_FEATURE
   <222> (17)..(17)
   <223> X=Q, R or K
<220>
   <221> MISC_FEATURE
   <222> (18)..(18)
   <223> X= S or F
<220>
   <221> MISC_FEATURE
   <222> (19)..(19)
   <223> X= M, I or L
<220>
   <221> MISC_FEATURE
   <222> (21)..(21)
   <223> X= I, V or M
<220>
   <221> MISC_FEATURE
   <222> (23)..(23)
   <223> X=A or T
<220>
   <221> MISC_FEATURE
   <222> (28)..(28)
   <223> X=E or D
<220>
   <221> MISC_FEATURE
   <222> (30)..(30)
   <223> X= D or G
<220>
   <221> MISC_FEATURE
   <222> (31)..(31)
   <223> X= V, F, A or I
<220>
   <221> MISC_FEATURE
   <222> (34)..(34)
   <223> X= S, N or R
<220>
   <221> MISC_FEATURE
   <222> (37)..(37)
   <223> X= V or I
<220>
   <221> MISC_FEATURE
   <222> (39)..(39)
   <223> X= A or T
<220>
   <221> MISC_FEATURE
   <222> (41)..(41)
   <223> X= K, Q or N
<220>
   <221> MISC_FEATURE
   <222> (48)..(48)
   <223> X= Q, G, R, H or E
<220>
   <221> MISC_FEATURE
   <222> (51)..(51)
   <223> X= S or L
<220>
   <221> MISC_FEATURE
   <222> (52)..(52)
   <223> X= L, Q or H
<220>
   <221> MISC_FEATURE
   <222> (54)..(54)
   <223> X= S, F or Y
<220>
   <221> MISC_FEATURE
   <222> (55)..(55)
   <223> X= G, K, S, N or R
<220>
   <221> MISC_FEATURE
   <222> (56)..(56)
   <223> X= D, H, S or N
<220>
   <221> MISC_FEATURE
   <222> (57)..(57)
   <223> X= A, H, M, E, G, S or T
<220>
   <221> MISC_FEATURE
   <222> (58)..(58)
   <223> X= S, V, P, T, N or D
<220>
   <221> MISC_FEATURE
   <222> (59)..(59)
   <223> X= I or L
<220>
   <221> MISC_FEATURE
   <222> (60)..(60)
   <223> X= H, S, K, N, Y or E
<220>
   <221> MISC_FEATURE
   <222> (61)..(61)
   <223> X= D or E
<220>
   <221> MISC_FEATURE
   <222> (62)..(62)
   <223> X= T, I or A
<220>
   <221> MISC_FEATURE
   <222> (63)..(63)
   <223> X= V or I
<220>
   <221> MISC_FEATURE
   <222> (64)..(64)
   <223> X= E, T, Q, R or K
<220>
   <221> MISC_FEATURE
   <222> (66)..(66)
   <223> X= L or V
<220>
   <221> MISC_FEATURE
   <222> (67)..(67)
   <223> X= I, T or L
<220>
   <221> MISC_FEATURE
   <222> (68)..(68)
   <223> X=I, M, F, Y or L
<220>
   <221> MISC_FEATURE
   <222> (72)..(72)
   <223> X= N, T, R, D or S
<220>
   <221> MISC_FEATURE
   <222> (73)..(73)
   <223> X= S, N, R, T or I
<220>
   <221> MISC_FEATURE
   <222> (75)..(75)
   <223> X= S or N
<220>
   <221> MISC_FEATURE
   <222> (76)..(76)
   <223> X= S or A
<220>
   <221> MISC_FEATURE
   <222> (77)..(77)
   <223> X= N, I or K
<220>
   <221> MISC_FEATURE
   <222> (78)..(78)
   <223> X= G, E or K
<220>
   <221> MISC_FEATURE
   <222> (79)..(79)
   <223> X= N, Y, D or T
<220>
   <221> MISC_FEATURE
   <222> (80)..(80)
   <223> X= V, K or I
<220>
   <221> MISC_FEATURE
   <222> (81)..(81)
   <223> X= T, A or I
<220>
   <221> MISC_FEATURE
   <222> (83)..(83)
   <223> X= S, L or T
<220>
   <221> MISC_FEATURE
   <222> (87)..(87)
   <223> X= E or V
<220>
   <221> MISC_FEATURE
   <222> (93)..(93)
   <223> X= E or K
<220>
   <221> MISC_FEATURE
   <222> (94)..(94)
   <223> X= K or R
<220>
   <221> MISC_FEATURE
   <222> (95)..(95)
   <223> x= N, T or S
<220>
   <221> MISC_FEATURE
   <222> (96)..(96)
   <223> X= I or F
<220>
   <221> MISC_FEATURE
   <222> (97)..(97)
   <223> X= K, N, A, or T
<220>
   <221> MISC_FEATURE
   <222> (101)..(101)
   <223> X= Q, K or E
<220>
   <221> MISC_FEATURE
   <222> (104)..(104)
   <223> X= V, I or K
<220>
   <221> MISC_FEATURE
   <222> (105)..(105)
   <223> X= H or R
<220>
   <221> MISC_FEATURE
   <222> (112)..(112)
   <223> X= N or Y
<220>
   <221> MISC_FEATURE
   <222> (113)..(113)
   <223> X= = T, S, P, L or A
<220>
   <221> MISC_FEATURE
   <222> (114)..(114)
   <223> X= = S or P
<400> 1
   Xaa Trp Xaa Xaa Val Xaa Xaa Asp Leu Xaa Xaa Ile Xaa Xaa Leu Xaa
   1               5                   10                  15
   Xaa Xaa Xaa His Xaa Asp Xaa Thr Leu Tyr Thr Xaa Ser Xaa Xaa His
               20                  25                  30
   Pro Xaa Cys Lys Xaa Thr Xaa Met Xaa Cys Phe Leu Leu Glu Leu Xaa
           35                  40                  45
   Val Ile Xaa Xaa Glu Xaa Xaa Xaa Xaa Xaa Xaa Xaa Xaa Xaa Xaa Xaa
       50                  55                  60
   Asn Xaa Xaa Xaa Leu Ala Asn Xaa Xaa Leu Xaa Xaa Xaa Xaa Xaa Xaa
   65                  70                  75                  80
   Xaa Glu Xaa Gly Cys Lys Xaa Cys Glu Glu Leu Glu Xaa Xaa Xaa Xaa
                   85                  90                  95
   Xaa Glu Phe Leu Xaa Ser Phe Xaa Xaa Ile Val Gln Met Phe Ile Xaa
               100                 105                 110
   Xaa Xaa
<210> 2
   <211> 114
   <212> PRT
   <213> Artificial Sequence
<220>
   <223> primate uinterleukin 15 consensus sequence
<220>
   <221> MISC_FEATURE
   <222> (1)..(1)
   <223> X = N or I
<220>
   <221> MISC_FEATURE
   <222> (4)..(4)
   <223> X = N or D
<220>
   <221> MISC_FEATURE
   <222> (10)..(10)
   <223> X = K or R
<220>
   <221> MISC_FEATURE
   <222> (11)..(11)
   <223> X = K or R
<220>
   <221> MISC_FEATURE
   <222> (13)..(13)
   <223> X = E or Q
<220>
   <221> MISC_FEATURE
   <222> (16)..(16)
   <223> X = I or T
<220>
   <221> MISC_FEATURE
   <222> (19)..(19)
   <223> X = M or I
<220>
   <221> MISC_FEATURE
   <222> (30)..(30)
   <223> X = D or G
<220>
   <221> MISC_FEATURE
   <222> (31)..(31)
   <223> X = V or I
<220>
   <221> MISC_FEATURE
   <222> (34)..(34)
   <223> X = S or R
<220>
   <221> MISC_FEATURE
   <222> (52)..(52)
   <223> X = L or H
<220>
   <221> MISC_FEATURE
   <222> (55)..(55)
   <223> X = G or N
<220>
   <221> MISC_FEATURE
   <222> (56)..(56)
   <223> X = D or N
<220>
   <221> MISC_FEATURE
   <222> (57)..(57)
   <223> X = A or T
<220>
   <221> MISC_FEATURE
   <222> (58)..(58)
   <223> X = S, N or D
<220>
   <221> MISC_FEATURE
   <222> (60)..(60)
   <223> X = H or N
<220>
   <221> MISC_FEATURE
   <222> (63)..(63)
   <223> X = V or I
<220>
   <221> MISC_FEATURE
   <222> (67)..(67)
   <223> X = I or L
<220>
   <221> MISC_FEATURE
   <222> (72)..(72)
   <223> X = N or R
<220>
   <221> MISC_FEATURE
   <222> (73)..(73)
   <223> X =S or I
<220>
   <221> MISC_FEATURE
   <222> (76)..(76)
   <223> X =S or A
<220>
   <221> MISC_FEATURE
   <222> (79)..(79)
   <223> X =N or T
<220>
   <221> MISC_FEATURE
   <222> (80)..(80)
   <223> X =V or I
<220>
   <221> MISC_FEATURE
   <222> (93)..(93)
   <223> X =E or K
<220>
   <221> MISC_FEATURE
   <222> (112)..(112)
   <223> X = N or Y
<220>
   <221> MISC_FEATURE
   <222> (113)..(113)
   <223> X = T or A
<400> 2
   Xaa Trp Val Xaa Val Ile Ser Asp Leu Xaa Xaa Ile Xaa Asp Leu Xaa
   1 5 10 15
   Gln Ser Xaa His Ile Asp Ala Thr Leu Tyr Thr Glu Ser Xaa Xaa His
   20 25 30
   Pro Xaa Cys Lys Val Thr Ala Met Lys Cys Phe Leu Leu Glu Leu Gln
   35 40 45
   Val Ile Ser Xaa Glu Ser Xaa Xaa Xaa Xaa Ile Xaa Asp Thr Xaa Glu
   50 55 60
   Asn Leu Xaa Ile Leu Ala Asn Xaa Xaa Leu Ser Xaa Asn Gly Xaa Xaa
   65 70 75 80
   Thr Glu Ser Gly Cys Lys Glu Cys Glu Glu Leu Glu Xaa Lys Asn Ile
   85 90 95
   Lys Glu Phe Leu Gln Ser Phe Val His Ile Val Gln Met Phe Ile Xaa
   100 105 110
   Xaa Ser
<210> 3
   <211> 114
   <212> PRT
   <213> Homo sapiens
<220>
   <221> VARIANT
   <222> (93)..(93)
   <223> X= E or K
<400> 3
   Asn Trp Val Asn Val Ile Ser Asp Leu Lys Lys Ile Glu Asp Leu Ile
   1               5                   10                  15
   Gln Ser Met His Ile Asp Ala Thr Leu Tyr Thr Glu Ser Asp Val His
               20                  25                  30
   Pro Ser Cys Lys Val Thr Ala Met Lys Cys Phe Leu Leu Glu Leu Gln
           35                  40                  45
   Val Ile Ser Leu Glu Ser Gly Asp Ala Ser Ile His Asp Thr Val Glu
       50                  55                  60
   Asn Leu Ile Ile Leu Ala Asn Asn Ser Leu Ser Ser Asn Gly Asn Val
   65                  70                  75                  80
   Thr Glu Ser Gly Cys Lys Glu Cys Glu Glu Leu Glu Xaa Lys Asn Ile
                   85                  90                  95
   Lys Glu Phe Leu Gln Ser Phe Val His Ile Val Gln Met Phe Ile Asn
               100                 105                 110
   Thr Ser
<210> 4
   <211> 61
   <212> PRT
   <213> Artificial Sequence
<220>
   <223> mammalian sushi domain consensus sequence
<220>
   <221> MISC_FEATURE
   <222> (3)..(3)
   <223> X= P or A
<220>
   <221> MISC_FEATURE
   <222> (5)..(5)
   <223> X= M or V
<220>
   <221> MISC_FEATURE
   <222> (7)..(7)
   <223> X= V or I
<220>
   <221> MISC_FEATURE
   <222> (13)..(13)
   <223> X= W, R or Q
<220>
   <221> MISC_FEATURE
   <222> (16)..(16)
   <223> X= S or N
<220>
   <221> MISC_FEATURE
   <222> (19)..(19)
   <223> X= L or V
<220>
   <221> MISC_FEATURE
   <222> (20)..(20)
   <223> X= Y, H or N
<220>
   <221> MISC_FEATURE
   <222> (26)..(26)
   <223> X= I or V
<220>
   <221> MISC_FEATURE
   <222> (39)..(39)
   <223> X= S or T
<220>
   <221> MISC_FEATURE
   <222> (41)..(41)
   <223> X=T or I
<220>
   <221> MISC_FEATURE
   <222> (45)..(45)
   <223> X=T or I
<220>
   <221> MISC_FEATURE
   <222> (45)..(45)
   <223> X= L or I
<220>
   <221> MISC_FEATURE
   <222> (48)..(48)
   <223> X= A or N
<220>
   <221> MISC_FEATURE
   <222> (51)..(51)
   <223> X= V or A
<220>
   <221> MISC_FEATURE
   <222> (53)..(53)
   <223> X= H or Y
<400> 4
   Cys Pro Xaa Pro Xaa Ser Xaa Glu His Ala Asp Ile Xaa Val Lys Xaa
   1 5 10 15
   Tyr Ser Xaa Xaa Ser Arg Glu Arg Tyr Xaa Cys Asn Ser Gly Phe Lys
   20 25 30
   Arg Lys Ala Gly Thr Ser Xaa Leu Xaa Glu Cys Val Xaa Asn Lys Xaa
   35 40 45
   Thr Asn Xaa Ala Xaa Trp Thr Thr Pro Ser Leu Lys Cys
   50 55 60
<210> 5
   <211> 64
   <212> PRT
   <213> Artificial Sequence
<220>
   <223> enlarged mammalian sushi domain consensus sequence
<220>
   <221> MISC_FEATURE
   <222> (1)..(1)
   <223> X= T, V or I
<220>
   <221> MISC_FEATURE
   <222> (5)..(5)
   <223> X= P or A
<220>
   <221> MISC_FEATURE
   <222> (7)..(7)
   <223> X= M or V
<220>
   <221> MISC_FEATURE
   <222> (9)..(9)
   <223> X= V or I
<220>
   <221> MISC_FEATURE
   <222> (15)..(15)
   <223> X= W, R or Q
<220>
   <221> MISC_FEATURE
   <222> (18)..(18)
   <223> X= S or N
<220>
   <221> MISC_FEATURE
   <222> (21)..(21)
   <223> X=L or V
<220>
   <221> MISC_FEATURE
   <222> (22)..(22)
   <223> X=Y, H or N
<220>
   <221> MISC_FEATURE
   <222> (28)..(28)
   <223> X= I or V
<220>
   <221> MISC_FEATURE
   <222> (41)..(41)
   <223> X= S or T
<220>
   <221> MISC_FEATURE
   <222> (43)..(43)
   <223> X= T or I
<220>
   <221> MISC_FEATURE
   <222> (47)..(47)
   <223> X= L or I
<220>
   <221> MISC_FEATURE
   <222> (50)..(50)
   <223> X= A or N
<220>
   <221> MISC_FEATURE
   <222> (53)..(53)
   <223> X= V or A
<220>
   <221> MISC_FEATURE
   <222> (55)..(55)
   <223> X= H or Y
<400> 5
   Xaa Thr Cys Pro Xaa Pro Xaa Ser Xaa Glu His Ala Asp Ile Xaa Val
   1               5                   10                  15
   Lys Xaa Tyr Ser Xaa Xaa Ser Arg Glu Arg Tyr Xaa Cys Asn Ser Gly
               20                  25                  30
   Phe Lys Arg Lys Ala Gly Thr Ser Xaa Leu Xaa Glu Cys Val Xaa Asn
           35                  40                  45
   Lys Xaa Thr Asn Xaa Ala Xaa Trp Thr Thr Pro Ser Leu Lys Cys Ile
       50                  55                  60
<210> 6
   <211> 61
   <212> PRT
   <213> Artificial Sequence
<220>
   <223> primate sushi domain consensus sequence
<220>
   <221> MISC_FEATURE
   <222> (3)..(3)
   <223> X= P or A
<220>
   <221> MISC_FEATURE
   <222> (5)..(5)
   <223> X= M or V
<220>
   <221> MISC_FEATURE
   <222> (13)..(13)
   <223> X= W, R or Q
<220>
   <221> MISC_FEATURE
   <222> (20)..(20)
   <223> X= Y or H
<220>
   <221> MISC_FEATURE
   <222> (26)..(26)
   <223> X= I or V
<220>
   <221> MISC_FEATURE
   <222> (51)..(51)
   <223> X= V or A
<220>
   <221> MISC_FEATURE
   <222> (53)..(53)
   <223> X= V or A
<400> 6
   Cys Pro Xaa Pro Xaa Ser Val Glu His Ala Asp Ile Xaa Val Lys Ser
   1               5                   10                  15
   Tyr Ser Leu Xaa Ser Arg Glu Arg Tyr Xaa Cys Asn Ser Gly Phe Lys
               20                  25                  30
   Arg Lys Ala Gly Thr Ser Ser Leu Thr Glu Cys Val Leu Asn Lys Ala
           35                  40                  45
   Thr Asn Xaa Ala Xaa Trp Thr Thr Pro Ser Leu Lys Cys
       50                  55                  60
<210> 7
   <211> 63
   <212> PRT
   <213> Artificial Sequence
<220>
   <223> enlarged primate sushi domain consensus sequence
<220>
   <221> MISC_FEATURE
   <222> (1)..(1)
   <223> X= V or I
<220>
   <221> MISC_FEATURE
   <222> (5)..(5)
   <223> X= P or A
<220>
   <221> MISC_FEATURE
   <222> (7)..(7)
   <223> X= M or V
<220>
   <221> MISC_FEATURE
   <222> (15)..(15)
   <223> X= W, R or Q
<220>
   <221> MISC_FEATURE
   <222> (22)..(22)
   <223> X= Y or H
<220>
   <221> MISC_FEATURE
   <222> (28)..(28)
   <223> X= I or V
<220>
   <221> MISC_FEATURE
   <222> (53)..(53)
   <223> X=V or A
<220>
   <221> MISC_FEATURE
   <222> (55)..(55)
   <223> X=H or Y
<400> 7
   Xaa Thr Cys Pro Xaa Pro Xaa Ser Val Glu His Ala Asp Ile Xaa Val
   1 5 10 15
   Lys Ser Tyr Ser Leu Xaa Ser Arg Glu Arg Tyr Xaa Cys Asn Ser Gly
   20 25 30
   Phe Lys Arg Lys Ala Gly Thr Ser Ser Leu Thr Glu Cys Val Leu Asn
   35 40 45
   Lys Ala Thr Asn Xaa Ala Xaa Trp Thr Thr Pro Ser Leu Lys Cys
   50 55 60
<210> 8
   <211> 61
   <212> PRT
   <213> Homo sapiens
<400> 8
   Cys Pro Pro Pro Met Ser Val Glu His Ala Asp Ile Trp Val Lys Ser
   1 5 10 15
   Tyr Ser Leu Tyr Ser Arg Glu Arg Tyr Ile Cys Asn Ser Gly Phe Lys
   20 25 30
   Arg Lys Ala Gly Thr Ser Ser Leu Thr Glu Cys Val Leu Asn Lys Ala
   35 40 45
   Thr Asn Val Ala His Trp Thr Thr Pro Ser Leu Lys Cys
   50 55 60
<210> 9
   <211> 64
   <212> PRT
   <213> Homo sapiens
<400> 9
   Ile Thr Cys Pro Pro Pro Met Ser Val Glu His Ala Asp Ile Trp Val
   1 5 10 15
   Lys Ser Tyr Ser Leu Tyr Ser Arg Glu Arg Tyr Ile Cys Asn Ser Gly
   20 25 30
   Phe Lys Arg Lys Ala Gly Thr Ser Ser Leu Thr Glu Cys Val Leu Asn
   35 40 45
   Lys Ala Thr Asn Val Ala His Trp Thr Thr Pro Ser Leu Lys Cys Ile
   50 55 60
<210> 10
   <211> 77
   <212> PRT
   <213> Artificial Sequence
<220>
   <223> mammalian sushi and domains consensus sequence
<220>
   <221> MISC_FEATURE
   <222> (1)..(1)
   <223> X= T, V or I
<220>
   <221> MISC_FEATURE
   <222> (5)..(5)
   <223> X= P or A
<220>
   <221> MISC_FEATURE
   <222> (7)..(7)
   <223> X= M or V
<220>
   <221> MISC_FEATURE
   <222> (9)..(9)
   <223> X= V or I
<220>
   <221> MISC_FEATURE
   <222> (15)..(15)
   <223> X= W, R or Q
<220>
   <221> MISC_FEATURE
   <222> (18)..(18)
   <223> X= S or N
<220>
   <221> MISC_FEATURE
   <222> (21)..(21)
   <223> X= L or V
<220>
   <221> MISC_FEATURE
   <222> (22)..(22)
   <223> X= Y, H or N
<220>
   <221> MISC_FEATURE
   <222> (28)..(28)
   <223> X= I or V
<220>
   <221> MISC_FEATURE
   <222> (41)..(41)
   <223> X= S or T
<220>
   <221> MISC_FEATURE
   <222> (43)..(43)
   <223> X= T or I
<220>
   <221> MISC_FEATURE
   <222> (47)..(47)
   <223> X= L or I
<220>
   <221> MISC_FEATURE
   <222> (50)..(50)
   <223> X=A or N
<220>
   <221> MISC_FEATURE
   <222> (53)..(53)
   <223> X= V or A
<220>
   <221> MISC_FEATURE
   <222> (55)..(55)
   <223> X= H or Y
<220>
   <221> MISC_FEATURE
   <222> (55)..(55)
   <223> X= H or Y
<220>
   <221> MISC_FEATURE
   <222> (68)..(68)
   <223> X=A, S or L
<220>
   <221> MISC_FEATURE
   <222> (70)..(70)
   <223> X= V, A, S or T
<220>
   <221> MISC_FEATURE
   <222> (71)..(71)
   <223> X= H or R
<220>
   <221> MISC_FEATURE
   <222> (72)..(72)
   <223> X= Q or H
<220>
   <221> MISC_FEATURE
   <222> (73)..(73)
   <223> X= R, S or K
<220>
   <221> MISC_FEATURE
   <222> (75)..(75)
   <223> X= A, V or P
<220>
   <221> MISC_FEATURE
   <222> (76)..(76)
   <223> X= P or S
<220>
   <221> MISC_FEATURE
   <222> (77)..(77)
   <223> X= P or T
<400> 10
   Xaa Thr Cys Pro Xaa Pro Xaa Ser Xaa Glu His Ala Asp Ile Xaa Val
   1               5                   10                  15
   Lys Xaa Tyr Ser Xaa Xaa Ser Arg Glu Arg Tyr Xaa Cys Asn Ser Gly
               20                  25                  30
   Phe Lys Arg Lys Ala Gly Thr Ser Xaa Leu Xaa Glu Cys Val Xaa Asn
           35                  40                  45
   Lys Xaa Thr Asn Xaa Ala Xaa Trp Thr Thr Pro Ser Leu Lys Cys Ile
       50                  55                  60
   Arg Asp Pro Xaa Leu Xaa Xaa Xaa Xaa Pro Xaa Xaa Xaa
   65                  70                  75
<210> 11
   <211> 77
   <212> PRT
   <213> Artificial Sequence
<220>
   <223> primate sushi and domains consensus sequence
<220>
   <221> MISC_FEATURE
   <222> (1)..(1)
   <223> X= V or I
<220>
   <221> MISC_FEATURE
   <222> (5)..(5)
   <223> X= P or A
<220>
   <221> MISC_FEATURE
   <222> (7)..(7)
   <223> X= M or V
<220>
   <221> MISC_FEATURE
   <222> (15)..(15)
   <223> X= W, R or Q
<220>
   <221> MISC_FEATURE
   <222> (22)..(22)
   <223> X= Y or H
<220>
   <221> MISC_FEATURE
   <222> (28)..(28)
   <223> X= I or V
<220>
   <221> MISC_FEATURE
   <222> (53)..(53)
   <223> X= V or A
<220>
   <221> MISC_FEATURE
   <222> (55)..(55)
   <223> X= H or Y
<220>
   <221> MISC_FEATURE
   <222> (68)..(68)
   <223> X= A, S or L
<220>
   <221> MISC_FEATURE
   <222> (70)..(70)
   <223> X= V, A or T
<220>
   <221> MISC_FEATURE
   <222> (71)..(71)
   <223> X= H or R
<220>
   <221> MISC_FEATURE
   <222> (75)..(75)
   <223> X= A or V
<400> 11
   Xaa Thr Cys Pro Xaa Pro Xaa Ser Val Glu His Ala Asp Ile Xaa Val
   1               5                   10                  15
   Lys Ser Tyr Ser Leu Xaa Ser Arg Glu Arg Tyr Xaa Cys Asn Ser Gly
               20                  25                  30
   Phe Lys Arg Lys Ala Gly Thr Ser Ser Leu Thr Glu Cys Val Leu Asn
           35                  40                  45
   Lys Ala Thr Asn Xaa Ala Xaa Trp Thr Thr Pro Ser Leu Lys Cys Ile
       50                  55                  60
   Arg Asp Pro Xaa Leu Xaa Xaa Gln Arg Pro Xaa Pro Pro
   65                  70                  75
<210> 12
   <211> 77
   <212> PRT
   <213> Homo sapiens
<400> 12
   Ile Thr Cys Pro Pro Pro Met Ser Val Glu His Ala Asp Ile Trp Val
   1 5 10 15
   Lys Ser Tyr Ser Leu Tyr Ser Arg Glu Arg Tyr Ile Cys Asn Ser Gly
   20 25 30
   Phe Lys Arg Lys Ala Gly Thr Ser Ser Leu Thr Glu Cys Val Leu Asn
   35 40 45
   Lys Ala Thr Asn Val Ala His Trp Thr Thr Pro Ser Leu Lys Cys Ile
   50 55 60
   Arg Asp Pro Ala Leu Val His Gln Arg Pro Ala Pro Pro
   65 70 75
<210> 13
   <211> 20
   <212> PRT
   <213> Artificial Sequence
<220>
   <223> peptidic linker
<400> 13
   Ser Gly Gly Ser Gly Gly Gly Gly Ser Gly Gly Gly Ser Gly Gly Gly
   1 5 10 15
   Gly Ser Leu Gln
   20
<210> 14
   <211> 20
   <212> PRT
   <213> Artificial Sequence
<220>
   <223> peptidic linker
<400> 14
   Ser Gly Gly Ser Gly Gly Gly Gly Ser Gly Gly Gly Ser Gly Gly Gly
   1 5 10 15
   Gly Ser Gly Gly
   20
<210> 15
   <211> 21
   <212> PRT
   <213> Artificial sequence
<220>
   <223> peptidic linker
<400> 15
   Ser Gly Gly Gly Ser Gly Gly Gly Gly Ser Gly Gly Gly Gly Ser Gly
   1 5 10 15
   Gly Gly Ser Leu Gln
   20
<210> 16
   <211> 211
   <212> PRT
   <213> Artificial Sequence
<220>
   <223> RLI2
<400> 16
   Ile Thr Cys Pro Pro Pro Met Ser Val Glu His Ala Asp Ile Trp Val
   1               5                   10                  15
   Lys Ser Tyr Ser Leu Tyr Ser Arg Glu Arg Tyr Ile Cys Asn Ser Gly
               20                  25                  30
   Phe Lys Arg Lys Ala Gly Thr Ser Ser Leu Thr Glu Cys Val Leu Asn
           35                  40                  45
   Lys Ala Thr Asn Val Ala His Trp Thr Thr Pro Ser Leu Lys Cys Ile
       50                  55                  60
   Arg Asp Pro Ala Leu Val His Gln Arg Pro Ala Pro Pro Ser Gly Gly
   65                  70                  75                  80
   Ser Gly Gly Gly Gly Ser Gly Gly Gly Ser Gly Gly Gly Gly Ser Gly
                   85                  90                  95
   Gly Asn Trp Val Asn Val Ile Ser Asp Leu Lys Lys Ile Glu Asp Leu
               100                 105                 110
   Ile Gln Ser Met His Ile Asp Ala Thr Leu Tyr Thr Glu Ser Asp Val
           115                 120                 125
   His Pro Ser Cys Lys Val Thr Ala Met Lys Cys Phe Leu Leu Glu Leu
       130                 135                 140
   Gln Val Ile Ser Leu Glu Ser Gly Asp Ala Ser Ile His Asp Thr Val
   145                 150                 155                 160
   Glu Asn Leu Ile Ile Leu Ala Asn Asn Ser Leu Ser Ser Asn Gly Asn
                   165                 170                 175
   Val Thr Glu Ser Gly Cys Lys Glu Cys Glu Glu Leu Glu Glu Lys Asn
               180                 185                 190
   Ile Lys Glu Phe Leu Gln Ser Phe Val His Ile Val Gln Met Phe Ile
           195                 200                 205
   Asn Thr Ser
       210
<210> 17
   <211> 211
   <212> PRT
   <213> Artificial Sequence
<220>
   <223> RLI1
<400> 17
   Ile Thr Cys Pro Pro Pro Met Ser Val Glu His Ala Asp Ile Trp Val
   1 5 10 15
   Lys Ser Tyr Ser Leu Tyr Ser Arg Glu Arg Tyr Ile Cys Asn Ser Gly
   20 25 30
   Phe Lys Arg Lys Ala Gly Thr Ser Ser Leu Thr Glu Cys Val Leu Asn
   35 40 45
   Lys Ala Thr Asn Val Ala His Trp Thr Thr Pro Ser Leu Lys Cys Ile
   50 55 60
   Arg Asp Pro Ala Leu Val His Gln Arg Pro Ala Pro Pro Ser Gly Gly
   65 70 75 80
   Ser Gly Gly Gly Gly Ser Gly Gly Gly Ser Gly Gly Gly Gly Ser Leu
   85 90 95
   Gln Asn Trp Val Asn Val Ile Ser Asp Leu Lys Lys Ile Glu Asp Leu
   100 105 110
   Ile Gln Ser Met His Ile Asp Ala Thr Leu Tyr Thr Glu Ser Asp Val
   115 120 125
   His Pro Ser Cys Lys Val Thr Ala Met Lys Cys Phe Leu Leu Glu Leu
   130 135 140
   Gln Val Ile Ser Leu Glu Ser Gly Asp Ala Ser Ile His Asp Thr Val
   145 150 155 160
   Glu Asn Leu Ile Ile Leu Ala Asn Asn Ser Leu Ser Ser Asn Gly Asn
   165 170 175
   Val Thr Glu Ser Gly Cys Lys Glu Cys Glu Glu Leu Glu Glu Lys Asn
   180 185 190
   Ile Lys Glu Phe Leu Gln Ser Phe Val His Ile Val Gln Met Phe Ile
   195 200 205
   Asn Thr Ser
   210

## Description

### Field of the Invention

The present invention relates to a new "combined pharmaceutical composition", and more specifically to a combination for treating cancer of a specific IL-15 superagonist and of an antibody antagonizing an immune pathway implicated in the inhibition of T cell activation as defined in the claims.

### Background

An adaptive immune response involves activation, selection, and clonal proliferation of two major classes of lymphocytes termed T cells and B cells. After encountering an antigen, T cells proliferate and differentiate into antigen-specific effector cells, while B cells proliferate and differentiate into antibody-secreting cells.

T cell activation is a multi-step process requiring several signaling events between the T cell and an antigen-presenting cell (APC). For T cell activation to occur, two types of signals must be delivered to a resting T cell.

The first type is mediated by the antigen-specific T cell receptor (TcR), and confers specificity to the immune response.

The second, costimulatory, type regulates the magnitude of the response and is delivered through accessory receptors on the T cell. These receptors comprise immunosuppressive receptors (e.g. CTL-A4, PD-1, or inhibitory KIRs) and co-stimulatory receptors (e.g. CD40, 4-1BB, OX-40 or glucocorticoid-induced TNFR-related protein (GITR)).

Therapeutic strategies have been developed on the inhibition of immunosuppressive receptors or on the activation of co-stimulatory receptors so as to potentiate the antitumoral response of the immune system.

WO 2012/175222 discloses an immunocytokine comprising (a) a conjugate, and (b) an antibody or a fragment thereof directly or indirectly linked by covalence to said conjugate, wherein said conjugate comprises (i) a polypeptide comprising the amino acid sequence of the interleukin 15 or derivatives thereof, and a polypeptide comprising the amino acid sequence of the sushi domain of the IL-15Ra or derivatives thereof. Xu et al. (Cancer Research, vol. 73, no. 10, pages 3075-3086, 2013) discloses that a single dose of a complex of an interleukin (IL)-15 superagonist mutant and a dimeric IL-15 receptor αSu/Fc fusion protein, but not IL-15 alone, eliminates 5T33P and MOPC-315P myeloma cells in the bone marrow of tumor-bearing mice.

Combined strategies are also envisaged actually. Now, the results are very hazardous depending on the tested compounds.

### Summary of the Invention

Now, the inventors show that a combination of their specific compound, named RLI, with an antibody antagonizing an immune pathway implicated in the inhibition of T cell activation results in a very high percentage of tumor remission, whereas such remission could not be envisaged in view of the tumor remission obtained with RLI or antagonists of immunosuppressive receptors alone.

Moreover, this synergic action was also obtained with a low-dose combination in which a low-dose of RLI was combined with a low-dose of anti-PD1. Surprisingly, and as compared to the previous combination, this combination has provided a strong and synergic inhibition of the tumor growth.

This strong synergy enable to envisage new therapies.

Consequently, the present invention relates to a combined pharmaceutical composition comprising:
1) a conjugate comprising (i) a polypeptide comprising the amino acid sequence of interleukin 15 or derivatives thereof having at least 10% of the activity of human IL-15 on the proliferation induction of kit225 cell line, and ii) a polypeptide comprising the amino acid sequence of the sushi domain of IL-15Rα or derivatives thereof having at least 10% of the binding activity of the sushi domain of human IL-15Rα to human IL-15; a polynucleotide coding therefore, or a vector comprising such a polynucleotide; and
2) an antibody antagonizing an immune pathway implicated in the inhibition of T cell activation, or a fragment thereof capable of reacting with the same antigen than its antibody counterpart, a polynucleotide coding therefore, or a vector comprising such a polynucleotide,
wherein said antibody is a PD-1/PD-L1/PD-L2 antagonist, wherein said antibody is antagonizing either by binding the PD-1 receptor or by binding the PD-L1 or PD-L2 ligand, wherein said conjugate and the antibody or fragment thereof are not linked.

In a second aspect, the invention relates to a pharmaceutical composition for use in treating cancer in a subject comprising
(A) a conjugate comprising (i) a polypeptide comprising the amino acid sequence of interleukin 15 or derivatives thereof having at least 10% of the activity of human IL-15 on the proliferation induction of kit225 cell line, and (ii) a polypeptide comprising the amino acid sequence of the sushi domain of IL-15Rα or derivatives thereof having at least 10% of the binding activity of the sushi domain of human IL-15Rα, to human IL-15, a polynucleotide coding therefore, or a vector comprising such a polynucleotide, wherein the use comprises simultaneously, separately, or sequentially administering (a) said pharmaceutical composition and (b) an antibody antagonizing an immune pathway implicated in the inhibition of T cell activation, or a fragment thereof which is capable of reacting with the same antigen than its antibody counterpart, a polynucleotide coding therefore, or a vector comprising such a polynucleotide, or
(B) an antibody antagonizing an immune pathway implicated in the inhibition of T cell activation, or a fragment thereof which is capable of reacting with the same antigen than its antibody counterpart, a polynucleotide coding therefore, or a vector comprising such a polynucleotide,
wherein the use comprises simultaneously, separately, or sequentially administering (a) said pharmaceutical composition and (b) a conjugate comprising (i) a polypeptide comprising the amino acid sequence of interleukin 15 or derivatives thereof having at least 10% of the activity of human IL-15 on the proliferation induction of kit225 cell line, and ii) a polypeptide comprising the amino acid sequence of the sushi domain of IL-15Rα or derivatives thereof having at least 10% of the binding activity of the sushi domain of human IL-15Rα to human IL-15, a polynucleotide coding therefore, or a vector comprising such a polynucleotide, and
wherein said antibody is a PD-1/PD-L1/PD-L2 antagonist,
wherein said antibody is antagonizing either by binding the PD-1 receptor or by binding the PD-L1 or PD-L2 ligand.

The pharmaceutical composition for use in treating cancer or the combined pharmaceutical composition of the present invention, are preferably adapted for simultaneous, separate, or sequential administration for use in treating cancer in a subject.

### Brief Description of the Drawings

Figure 1 shows the injection protocol used in the mouse cancer model with BIOXELL anti-PD1
Figure 2 shows the TILs presence in the CT26 model.
Figure 3 shows the Anti-PD1 (RPMI1-14) and RLI combination therapy in mice.
Figure 4 shows the mice survival for anti-PD1 / RLI combination treatment.
Figure 5 shows the injection protocol used in the mouse cancer model.
Figure 6 shows the anti-PD1 (mBAT) and RLI combination therapy in mice.

### Detailed Description

### Conjugate

The term "interleukin 15" in its general meaning in the art and refers to a cytokine with structural similarity to IL-2 (GRABSTEIN et al., Science, vol.264(5161), p:965-968, 1994). This cytokine is also known as IL-15, IL15 or MGC9721. This cytokine and IL-2 share many biological activities and they were found to bind common hematopoietin receptor subunits. Thus, they may compete for the same receptor, negatively regulating each other's activity. It has been established that IL-15 regulates T and natural killer cells activation and proliferation, and that the number of CD8+ memory cells is shown to be controlled by a balance between this cytokine and IL2. IL-15 activity can be measured by determining its proliferation induction on kit225 cell line (HORI et al., Blood, vol.70(4), p:1069-72, 1987), as disclosed in the Examples.

Said IL-15 or derivatives thereof have at least 10% of the activity of human interleukin-15 on the proliferation induction of kit225 cell line, preferably at least 25% and more preferably at least 50%.

Said interleukin 15 is a mammalian interleukin 15, preferably a primate interleukin 15, and more preferably a human interleukin 15.

Mammalian interleukin 15 can be simply identified by the skilled person. As an example, one can cite Interleukin 15 from *Sus scrofa* (Accession number ABF82250), from *Rattus norvegicus* (Accession number NP_037261), from *Mus musculus* (Accession number NP_032383), *from Bos Taurus* (Accession number NP _776515), from *Oryctolagus cuniculus* (Accession number NP_001075685), from *Ovies aries* (Accession number NP_001009734), from *Felis catus* (Accession number NP_001009207), from *Macaca fascicularis* (Accession number BAA19149), from *Homo sapiens* (Accession number NP_000576), from *Macaca Mulatta* (Accession number NP_001038196), from *Cavia porcellus* (Accession number NP_001166300), or from *Chlorocebus sabaeus* (Accession number ACI289).

As used herein, the term "mammalian interleukin 15" refers to the consensus sequence SEQ ID n°1.

Primate Interleukin 15 can be simply identified by the skilled person. As an example, one can cite Interleukin 15 from *Sus scrofa* (Accession number ABF82250), from *Oryctolagus cuniculus* (Accession number NP_001075685), from *Macaca fascicularis* (Accession number BAA19149), from *Homo sapiens* (Accession number NP_000576), from *Macaca Mulatta* (Accession number NP_001038196), or from *Chlorocebus sabaeus* (Accession number ACI289).

As used herein, the term "primate interleukin 15" refers to the consensus sequence SEQ ID n°2.

Human interleukin 15 can be simply identify by the skilled person and refers to the amino acids sequence SEQ ID n° 3.

As used herein, the term "interleukin 15 derivatives" refers to an amino acid sequence having a percentage of identity of at least 92.5 % (i.e. corresponding to about 10 amino acids substitutions) with an amino acid sequence selected in the group consisting of SEQ ID n°: 1, SEQ ID n°2 and SEQ ID n°3, preferably of at least 96 % (i.e. corresponding to about 5 amino acids substitutions), and more preferably of at least 98.5% (i.e. corresponding to about 2 amino acids substitutions) or of at least 99% i.e. corresponding to about 1 amino acid substitution). Such derivatives can be simply identified by the skilled person in view of its personal knowledge and of the teaching of the present patent application. As an example of such derivatives, one can cite those described in the International Patent Application PCT WO 2009/135031. It will also be understood that natural amino acids may be replaced by chemically modified amino acids. Typically, such chemically modified amino acids increase the polypeptide half life.

As used herein, "percentage of identity" between two amino acids sequences, means the percentage of identical amino-acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously aligned according to the best alignment; this comparison is realized on segments of comparison in order to identify and compare the local regions of similarity. The best sequences alignment to perform comparison can be realized, beside by a manual way, by using the global homology algorithm developed by SMITH and WATERMAN (Ad. App. Math., vol.2, p:482, 1981), by using the local homology algorithm developed by NEDDLEMAN and WUNSCH (J. Mol. Biol., vol.48, p:443, 1970), by using the method of similarities developed by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p: 1792, 2004). To get the best local alignment, one can preferably use the BLAST software with the BLOSUM 62 matrix. The identity percentage between two sequences of amino acids is determined by comparing these two sequences optimally aligned, the amino acids sequences being able to encompass additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

Preferably, the interleukin 15 derivatives are IL-15 agonist or superagonist. One skilled in the art can simply identify an IL-15-agonist or -superagonist. As a example of IL-15-agonist or -superagonist, one can cite the ones disclosed in the International patent application WO 2005/085282 or in ZHU et al. (J. Immunol., vol. 183(6), p:3598-607, 2009).

Still preferably, said IL-15 agonist or superagonist is selected in the group comprising/consisting of L45D, L45E, S51D, L52D, N72D, N72E, N72A, N72S, N72Y and N72P (in reference to sequence of human IL-15, SEQ ID n°3).

As used herein the term "the sushi domain of IL-15Rα" has its general meaning in the art and refers to a domain beginning at the first cysteine residue (C1) after the signal peptide of IL-15Rα, and ending at the fourth cysteine residue (C4) after said signal peptide. Said sushi domain corresponding to a portion of the extracellular region of IL-15Rα is necessary for its binding to IL-15 (WEI et al., J. Immunol., vol.167(1), p:277-282, 2001).

Said sushi domain of IL-15Rα or derivatives thereof has at least 10% of the binding activity of the sushi domain of human IL-15Rα to human interleukin-15, preferably at least 25% and more preferably at least 50%. Said binding activity can be simply determined by the method disclosed in WEI et al. (abovementioned, 2001).

Said sushi domain of the IL-15Rα is the sushi domain of a mammalian IL-15Rα, preferably the sushi domain of a primate IL-15Rα and more preferably the sushi domain of the human IL-15Rα.

The sushi domain of a mammalian IL-15Rα can be simply identified by the skilled person. As an example, one can cite the sushi domain of a IL-15Rα from *Rattus norvegicus* (Accession number XP_002728555), from *Mus musculus* (Accession number EDL08026), from *Bos Taurus* (Accession number XP_002692113), from *Oryctolagus cuniculus* (Accession number XP_002723298), from *Macaca fascicularis* (Accession number ACI42785), from *Macaca nemestrina* (Accession number ACI42783), from *Homo sapiens* (Accession number Q13261.1), from *Macaca Mulatta* (Accession number NP_001166315), *Pongo abelii* (Accession number XP_002820541), *Cercocebus torquatus* (Accession number ACI42784), *Callithrix jacchus* (Accession number XP_002750073), or from *Cavia porcellus* (Accession number NP_001166314).

As used herein, the term "sushi domain of a mammalian IL-15Rα" refers to the consensus sequence SEQ ID n°4.

Preferably, the polypeptide comprising the amino acid sequence of the sushi domain of a mammalian IL-15Rα refers to the consensus sequence SEQ ID n°5.

The sushi domain of a primate IL-15Rα can be simply identified by the skilled person. As an example, one can cite sushi domains of IL-15Rα from *Oryctolagus cuniculus,* from *Macaca fascicularis*, from *Macaca nemestrina*, from *Homo sapiens,* from *Macaca Mulatta*, *Pongo abelii*, *Cercocebus torquatus*, or *Callithrix jacchus.*

As used herein, the term "sushi domain of a primate IL-15Rα" refers to the consensus sequence SEQ ID n°6.

Preferably, the polypeptide comprising the amino acid sequence of the sushi domain of a primate IL-15Rα refers to the consensus sequence SEQ ID n°7.

The sushi domain of human IL-15Rα can be simply identified by the skilled person and refers to the amino acids sequence SEQ ID n° 8.

Preferably, the polypeptide comprising the amino acid sequence of the sushi domain of human IL-15Rα refers to SEQ ID n° 9.

As used herein, the term "derivatives of the sushi domain of the IL-15Rα" refers to an amino acid sequence having a percentage of identity of at least 92 % (i.e. corresponding to about 5 amino acids substitutions) with an amino acid sequence selected in the group consisting of SEQ ID n°: 4, SEQ ID n°5, SEQ ID n°6, SEQ ID n°: 7, SEQ ID n°8, and SEQ ID n°9, preferably of at least 96 % (i.e. corresponding to about 2 amino acids substitutions), and more preferably of at least 98% (i.e. corresponding to about 1 amino acids substitutions). Such derivatives comprise the four cysteine residues of the sushi domain of L-15Rα and can be simply identified by the skilled person in view of his/her general knowledge and of the teaching of the present patent application. It will also be understood that natural amino acids may be replaced by chemically modified amino acids. Typically, such chemically modified amino acids enable to increase the polypeptide half life.

According to a preferred embodiment, the conjugate comprises (ii) a polypeptide comprising the amino acid sequence of the sushi and hinge domains of IL-15Rα or derivatives thereof.

The IL-15Rα hinge domain is defined as the amino acid sequence that begins at the first amino residue after the sushi domain and that ends at the last amino acid residue before the first potential site of glycosylation. In human IL-15Rα, the amino acid sequence of the hinge region consists of the fourteen amino acids which are located after the sushi domain of this IL- 15Ralpha, in a C-terminal position relative to said sushi domain, i.e., said IL- 15Ralpha hinge region begins at the first amino acid after said (C4) cysteine residue, and ends at the fourteenth amino acid (counting in the standard "from N- terminal to C-terminal" orientation).

Said sushi and hinge domains of IL-15Rα are the sushi and hinge domains of a mammalian IL-15Rα, preferably the sushi and hinge domains of a primate IL-15Rα and more preferably the sushi and hinge domains of the human IL-15Rα.

The amino acid sequence of the sushi and hinge domains of a mammalian IL-15Rα can be simply identified by the skilled person. As used herein, the term "sushi and hinge domains of a mammalian IL-15Rα" refers to the consensus sequence SEQ ID n°10.

The amino acid sequence of the sushi and hinge domains of a primate IL-15Rα can be simply identified by the skilled person. As used herein, the term "sushi and hinge domains of a primate IL-15Rα" refers to the consensus sequence SEQ ID n°11.

The amino acid sequence of the sushi and hinge domains of human IL-15Rα can be simply identified by the skilled person. As used herein, the term "sushi and hinge domains of human IL-15Rα" refers to the consensus sequence SEQ ID n°12.

As used herein, the term "derivatives of the sushi and hinge domains of IL-15Rα" refers to an amino acid sequence having a percentage of identity of at least 93 % (i.e. corresponding to about 5 amino acids substitutions) with an amino acid sequence selected in the group consisting of SEQ ID n°: 10, SEQ ID n°11, and SEQ ID n°12, preferably of at least 97 % (i.e. corresponding to about 2 amino acids substitutions), and more preferably of at least 98% (i.e. corresponding to about 1 amino acids substitution). Such derivatives comprise the four cysteine residues of the sushi domain of L-15Rα and can be simply identified by the skilled person in view of its general knowledge and of the teaching of the present patent application. It will also be understood that natural amino acids may be replaced by chemically modified amino acids. Typically, such chemically modified amino acids enable to increase the polypeptide half life.

Both polypeptides i) and ii) of the conjugate may be linked non-covalently such as in the complex disclosed in Patent US 8,124,084 B2. Said conjugate or complex can be simply obtained by providing a suitable amount of the polypeptide i), providing a suitable amount of the polypeptide ii), admixing both polypeptides under suitable pH and ionic conditions for a duration sufficient to allow complex (i.e. conjugate) formation, and optionally concentrating or purifying said complex. The polypeptides of the complex (i.e. conjugate) can be formed, for example, using a peptide synthesizer according to standard methods; by expressing each polypeptide separately in a cell or cell extract, then isolating and purifying the polypeptide. Optionally, the therapeutic polypeptide complex of the invention can be formed by expressing both polypeptides i) and ii) in the same cell or cell extract, then isolating and purifying the complexes, for example, using chromatographic techniques, such as affinity chromatography with antibodies to the lymphokine portion, the lymphokine receptor portion, or to the complex.

Both polypeptides i) and ii) of the conjugate may be also covalently linked using bifunctional protein coupling agents or in a fusion protein.

Bifunctional protein coupling agents are well known from the skilled person such as methods using them, and include, as examples, N-succinimidyl (2-pyridyldithio) propionate (SPDP), succinimidyl (N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene).

The term "fusion protein" refers to a protein created through the joining of two or more genes which originally coded for separate proteins. It is also known as a chimeric protein. Translation of this fusion gene results in a single polypeptide with functional properties deriving from each of the original proteins. Recombinant fusion proteins are created artificially by recombinant DNA technology for use in biological research or therapeutics. A recombinant fusion protein is a protein created through genetic engineering of a fusion gene. This typically involves removing the stop codon from a cDNA sequence coding for the first protein, then appending the cDNA sequence of the second protein in frame through ligation or overlap extension PCR. That DNA sequence will then be expressed by a cell as a single protein. The protein can be engineered to include the full sequence of both original proteins, or only a portion of either.

In a preferred embodiment, the conjugate is a fusion protein.

The amino acid sequence of interleukin 15 or derivatives thereof can be in a C-terminal or in an N-terminal position relative to the amino acid sequence of the sushi domain of IL-15Rα or derivatives thereof. Preferably, the amino acid sequence of the interleukin 15 or derivatives thereof is in a C-terminal position relative to the amino acid sequence of the sushi domain of IL-15Rα or derivatives thereof.

The amino acid sequence of interleukin 15 or derivatives thereof and the amino acid sequence of the sushi domain of IL-15Rα or derivatives thereof may be separated by a first "linker" amino acid sequence. Said first "linker" amino acid sequence may be of a length sufficient to ensure that the fusion protein form proper secondary and tertiary structures.

The length of the first linker amino acid sequence may vary without significantly affecting the biological activity of the fusion protein. Typically, the first linker amino acid sequence comprises at least one, but less than 30 amino acids e.g., a linker of 2-30 amino acids, preferably of 10-30 amino acids, more preferably of 15-30 amino acids, still more preferably of 15-25 amino acids, most preferably of 18-22 amino acids.

Preferred linker amino acid sequences are those which allow the conjugate to adopt a proper conformation (i.e., a conformation allowing a proper signal transducing activity through the IL-15Rbeta/gamma signaling pathway).

The most suitable first linker amino acid sequences (1) will adopt a flexible extended conformation, (2) will not exhibit a propensity for developing ordered secondary structure which could interact with the functional domains of fusion proteins, and (3) will have minimal hydrophobic or charged character which could promote interaction with the functional protein domains.

Preferably, the first linker amino acid sequence comprises near neutral amino acids selected in the group comprising Gly (G), Asn (N), Ser (S), Thr (T), Ala (A), Leu (L), and Gln (Q), most preferably in the group comprising Gly (G), Asn (N), and Ser (S).

Examples of linker sequences are described in U.S. Pat. Nos. 5,073,627 and 5,108,910.

Illustrative flexible linkers that are more particularly suitable for the present invention include those coded by the sequences of SEQ ID NO: 13 (SGGSGGGGSGGGSGGGGSLQ), SEQ ID n°14 (SGGSGGGGSGGGSGGGGSGG) or SEQ ID n°15 (SGGGSGGGGSGGGGSGGGSLQ).

Still preferably, the conjugate is a fusion protein having the sequence SEQ ID n°16 or SEQ ID n°17.

### immunosuppressive receptor antagonist antibody

The term "antibody" refers to an immunoglobulin molecule corresponding to a tetramer comprising four polypeptide chains, two identical heavy (H) chains (about 50-70 kDa when full length) and two identical light (L) chains (about 25 kDa when full length) inter-connected by disulfide bonds. Light chains are classified as kappa and lambda. Heavy chains are classified as gamma, mu, alpha, delta, or epsilon, and define the antibody's isotype as IgG, IgM, IgA, IgD, and IgE, respectively. Each heavy chain is comprised of a N-term heavy chain variable region (abbreviated herein as HCVR) and a heavy chain constant region. The heavy chain constant region is comprised of three domains (CH1, CH2, and CH3) for IgG, IgD, and IgA; and 4 domains (CHI, CH2, CH3, andCH4) for IgM and IgE. Each light chain is comprised of a N-term light chain variable region (abbreviated herein as LCVR) and a light chain constant region. The light chain constant region is comprised of one domain, CL. The HCVR and LCVR regions can be further subdivided into regions of hypervariability, termed complementarity determining regions (CDRs), interspersed with regions that are more conserved, termed framework regions (FR). Each HCVR and LCVR is composed of three CDRs and four FRs, arranged from amino-terminus to carboxy-terminus in the following order: FR1, CDR1, FR2, CDR2, FR3, CDR3, FR4. The assignment of amino acids to each domain is in accordance with well-known conventions. The functional ability of the antibody to bind a particular antigen depends on the variable regions of each light/heavy chain pair, and is largely determined by the CDRs.

The term "antibody", as used herein, refers to a monoclonal antibody *per se.* A monoclonal antibody can be a human antibody, chimeric antibody and/or humanized antibody.

Advantageously, the term antibody refers to an IgG, such as IgG1, IgG2 (IgG2a or IgG2b), IgG3 and IgG4. Preferably, the term antibody refers to IgG1 or IgG2, and more preferably to IgG2a.

"Chimeric antibody" means an antibody that is composed of variables regions from a murine immunoglobulin and of constant regions of a human immunoglobulin. This alteration consists simply of substituting the constant region of a human antibody with the murine constant region, thus resulting in a human/murine chimera which may have sufficiently low immunogenicity to be acceptable for pharmaceutical use. A number of methods for producing such chimeric antibodies have yet been reported, thus forming part of the general knowledge of the skilled artisan (See, e.g., U.S. Pat. No. 5,225,539).

"Humanized antibody" means an antibody that is composed partially or fully of amino acid sequences derived from a human antibody germline by altering the sequence of an antibody having non-human complementarity determining regions (CDR). This humanization of the variable region of the antibody and eventually the CDR is made by techniques that are by now well known in the art. As an example, British Patent Application GB 2188638A and US Patent No. 5,585,089 disclose processes wherein recombinant antibodies are produced where the only portion of the antibody that is substituted is the complementarity determining region, or "CDR". The CDR grafting technique has been used to generate antibodies which consist of murine CDRs, and human variable region framework and constant regions (See. e. g., RIECHMANN et al., Nature , vol.332, p: 323-327, 1988). These antibodies retain the human constant regions that are necessary for Fc dependent effector function, but are much less likely to evoke an immune response against the antibody. As an example, the framework regions of the variable regions are substituted by the corresponding human framework regions leaving the non-human CDR substantially intact, or even replacing the CDR with sequences derived from a human genome. Fully human antibodies are produced in genetically modified mice whose immune systems have been altered to correspond to human immune systems. As mentioned above, it is sufficient for use in the methods of the invention, to employ an immunologically specific fragment of the antibody, including fragments representing single chain forms.

A humanized antibody again refers to an antibody comprising a human framework, at least one CDR from a non-human antibody, and in which any constant region present is substantially identical to a human immunoglobulin constant region, i. e., at least about 85 or 90%, preferably at least 95% identical. Hence, all parts of a humanized antibody, except possibly the CDRs, are substantially identical to corresponding parts of one or more native human immunoglobulin sequences. For example, a humanized immunoglobulin would typically not encompass a chimeric mouse variable region/human constant region antibody. As an example, the design of humanized immunoglobulins may be carried out as follows: when an amino acid falls under the following category, the framework amino acid of a human immunoglobulin to be used (acceptor immunoglobulin) is replaced by a framework amino acid from a CDR-providing non-human immunoglobulin (donor immunoglobulin) : (a) the amino acid in the human framework region of the acceptor immunoglobulin is unusual for human immunoglobulin at that position, whereas the corresponding amino acid in the donor immunoglobulin is typical for human immunoglobulin at that position ; (b) the position of the amino acid is immediately adjacent to one of the CDRs ; or (c) any side chain atom of a framework amino acid is within about 5-6angstroms (center-to-center) of any atom of a CDR amino acid in a three dimensional immunoglobulin model (CO M. S. et al., Proc. Natl. Acad. Sci. USA, vol.88, p:2869, 1991). When each of the amino acids in the human framework region of the acceptor immunoglobulin and a corresponding amino acid in the donor immunoglobulin is unusual for human immunoglobulin at that position, such an amino acid is replaced by an amino acid typical for human immunoglobulin at that position.

The term "antibody fragment" as used herein refers to antibody fragment capable of reacting with the same antigen than its antibody counterpart. Such fragments can be simply identified by the skilled person and comprise, as an example, F_{ab} fragment (e.g., by papain digestion), F_{ab}' fragment (e.g., by pepsin digestion and partial reduction), F(_{ab}')₂ fragment (e.g., by pepsin digestion), F_{acb} (e.g., by plasmin digestion), F_{d} (e.g., by pepsin digestion, partial reduction and reaggregation), and also scFᵥ (single chain Fv; e.g., by molecular biology techniques) fragment are encompassed by the invention.

Such fragments can be produced by enzymatic cleavage, synthetic or recombinant techniques, as known in the art and/or as described herein. Antibodies can also be produced in a variety of truncated forms using antibody genes in which one or more stop codons have been introduced upstream of the natural stop site. For example, a combination gene encoding a F(_{ab}')₂ heavy chain portion can be designed to include DNA sequences encoding the CH₁ domain and/or hinge region of the heavy chain. The various portions of antibodies can be joined together chemically by conventional techniques, or can be prepared as a contiguous protein using genetic engineering techniques.

Preferably, said antibody fragment is a scFv fragment.

The term "antibody antagonizing an immune pathway implicated in the inhibition of T cell activation" refers to an antibody antagonizing the immunosuppressive receptor PD-1 either by binding this receptor or by binding its ligand, thus promoting immune activation by preventing downregulation signals. It is further disclosed that another "antibody antagonizing an immune pathway implicated in the inhibition of T cell activation" is an antibody antagonizing the immunosuppressive receptor CTL-A4 or inhibitory KIRs either by binding this receptor or by binding its ligand, thus promoting immune activation by preventing downregulation signals. As an example of immunosuppressive receptor antagonist antibody, one can cite antibodies corresponding to CTL-A4, PD-1/PD-L1, PD-1/PD-L2, inhibitory KIRs, CD276, VTCN1, BTLA/HVEM, LAG3, HAVCR2 and ADORA2A antagonist, preferably PD-1/PD-L1 antibodies.
CTL-A4 (Cytotoxic Lymphocyte Associated Antigen, also designated CD 152) was discovered in 1987 (BRUNET et al., Nature, vol.328, p:267-270, 1987). The role of CTL-A4 is primarily to inhibit T cell activation and this was shown in CTL-A4 deficient mice suffering from massive lymphoproliferation (CHAMBERS et al., Immunity, vol.7, p:8855-8959, 1997). Now, the blockage of CTL-A4 has been shown to enhance T cell responses *in vitro* (WALUNAS et al., Immunity, vol.1, p:405-413, 1994) and *in vivo* (KEARNEY, J. Immunol, vol.155, p:1032-1036, 1995) and also to increase antitumour immunity (LEACH, Science, vol.271, p:1734-1736, 1996). As an example of antibodies corresponding to CTL-A4 antagonists, one can cite ipilimumab (also referred to as MDX-010 and 10D1, available from MEDAREX, and marketed as YERVOY™ by BRISTOL-MYERS SQUIBB COMPANY) disclosed in WO 01/14424, ticilimumab (also known as 11.2.1 and CP-675,206) disclosed in WO 00/37504, and also the CTL-A4 antibodies disclosed in International patent applications WO 98/42752, WO 01/14424, WO 2004/035607, and WO 2012/120125, in EP 1212422 and EP 1262193, in U.S. Patent Nos. US 5,81 1,097, US 5,855,887, US 5,977,318, US 6,051,227, US 6,207,156, US 6,682,736, US 6,984,720, US 7, 109,003, and US 7,132,281.

Programmed Cell Death 1 also known as PD-1 (also referred to as PDCD1 or CD279) is a ∼55kD type I membrane glycoprotein. PD-1 is a receptor of the CD28 costimulatory gene family, which is moderately expressed on naive T, B and NK cells and up-regulated by T/B cell receptor signaling on lymphocytes, monocytes and myeloid cells. PD-1 has two known ligands with distinct expression profiles, PD-L1 (B7-H1), which is widely expressed -i.e. on naive lymphocytes on activated B and T cells, monocytes and dendritic cells -, and PD-L2 (B7-DC), whose expression is restricted -i.e. on activated dendritic cells, macrophages and monocytes and on vascular endothelial cells-. In several murine syngeneic tumor models, blockade of either PD-1 or PD-L1 significantly inhibited tumor growth or induced complete regression. Thus, the PD-1 is recognized as an important player in immune regulation and the maintenance of peripheral tolerance. As an example of antibodies corresponding to PD-1/PD-L1 /PD-L2 antagonists, one can cite nivolumab (also known as BMS-936558 or MDX1106; anti-PD-1 antibody, BRISTOL-MYERS SQUIBB) is disclosed in WO 2006/121168, Merck 3745 (also known as MK-3475 or SCH-900475, is an anti-PD- 1 antibody) is disclosed in WO 2009/114335, CT-01 1 (also known as hBAT or hBAT-1, anti-PD-1 antibody) is disclosed in WO 2009/101611, lambrolizumab is disclosed in WO2008/156712, AMP514 which is disclosed in WO2010/027423, WO2010/027827, WO2010/027828, and WO2010/098788, and also the antibodies disclosed in International patent applications WO 2004/056875, WO 2006/056875, WO 2008/083174, WO 2010/029434, WO 2010/029435, WO 2010/036959, WO 2010/089411, WO 2011/110604, WO 2012/135408, and WO 2012/145493. Said PD-1 antagonist may correspond to an anti-PD-L1 antibody such as MDX- 1 105 (also known as BMS-936559, anti-PD-L1 antibody) disclosed in WO 2007/005874, or YW243.55.S70 (also known as MPDL3280A or RG7446; anti-PD-L1 antibody) disclosed in WO 2010/077634.

Killer-cell immunoglobulin-like receptors (KIRs), are a family of cell surface proteins found on important cells of the immune system called natural killer (NK) cells. They regulate the killing function of these cells by interacting with MHC class I molecules, which are expressed on all cell types. This interaction allows them to detect virally infected cells or tumor cells that have a characteristic low level of Class I MHC on their surface. Most KIRs are inhibitory, meaning that their recognition of MHC suppresses the cytotoxic activity of their NK cell. Only a limited number of KIRs have the ability to activate cell.

Inhibitory KIRs have a long cytoplasmic tail containing Immunoreceptor Tyrosine-based Inhibitory Motif (ITIM), which transduce inhibitory signals to the NK cell upon engagement of their MHC class I ligands. The known inhibitory KIRs include members of the KIR2DL and KIR3DL subfamilies comprising KIR2DL1, KIR2DL2, KIR2DL3, KIR2DL4, KIR2DL5A, KIR2DL5B,KIR3DL1, KIR3DL2, and KIR3DL3. As an example of antibodies corresponding to inhibitory KIRs antagonists, one can cite the antibody 1-7F9 disclosed in WO 2006/003179.

The B7 ligand family members are known to have strong immunoregulatory activity by immune cells and more recently by tumor cells, this family comprises CD276 and VTCN1.

CD276 (Cluster of Differentiation 276) (also known as B7H3, B7-H3; B7RP-2; 4Ig-B7-H3) is a type I transmembrane protein from the B7 ligand family, which was first identified in dendritic cells and activated T-cells. CD276 is expressed by some solid tumours and is thought to participate in the regulation of T-cell-mediated immune response. More especially, it seems that CD276 downregulates T-helper immune response and suppresses immunity (SUH W. K. et al., 2003. Nat Immunol 4(9): 899-906). As an example of CD276 antagonist, one can cite the antibody 8H9 disclosed in the patent application US 2005/0169932 and WO 2008/116219, and other antibodies such as MGA271 disclosed in WO 2011/109400.

VTCN1 (V-set domain-containing T-cell activation inhibitor 1), also known as B7X; B7H4; B7S1; B7-H4, is also a member of the B7 family.VTCN1 is thought to play a role in inhibitory regulation of T cell response and studies have shown that high levels of this protein have been correlated with tumor progression. As an example of VTCN1 antagonists, one can cite the antibodies disclosed in WO 2009/073533.

BTLA (B- and T-lymphocyte attenuator), also known as CD272, is induced during activation of T cells, and remains expressed on Th1 cells but not Th2 cells. BTLA displays T-Cell inhibition via interaction with tumor necrosis factor (receptor), member 14 (TNFRSF14), also known as herpes virus entry mediator (HVEM), TR2; ATAR; HVEA; CD270; LIGHTR. TNFRSF14 was identified as a cellular mediator of herpes simplex virus (HSV) entry. The cytoplasmic region of this receptor was found to bind to several TRAF family members, which may mediate the signal transduction pathways that activate the immune response. Finally, the BTLA/HVEM complexes negatively regulate T-cell immune responses. As an example of BTLA/HVEM antagonist, one can cite the antibodies disclosed in WO 2008/076560, WO 2010/106051, and WO 2011/014438.

LAG3 (Lymphocyte-activation gene 3, also known as CD223) belongs to immunoglobulin (Ig) superfamily and contains 4 extracellular Ig-like domains. As an example of LAG3 antagonists, one can cite the antibodies disclosed in WO 2010/019570.

HAVCR2 (Hepatitis A virus cellular receptor 2, also known as Tim-3, KIM-3; TIMD3; Tim-3; and TIMD-3) is a Th1-specific cell surface protein belonging to the immunoglobulin superfamily. HAVCR2 regulates macrophage activation and inhibits Th1-mediated auto- and alloimmune responses, thus promoting immunological tolerance. As an example of HAVCR2 antagonists, one can cite the antibodies disclosed in WO 2013/006490A.

ADORA2A (adenosine A_{2A} receptor, also known as A2aR, RDC8; or ADORA2) belongs to the guanine nucleotide-binding protein (G protein)-coupled receptor (GPCR) superfamily, which is subdivided into classes and subtypes. This protein plays an important role in many biological functions, such as cardiac rhythm and circulation, cerebral and renal blood flow, immune function, pain regulation, and sleep. It has been implicated in pathophysiological conditions such as inflammatory diseases and neurodegenerative disorders.

Said antibody or fragment thereof and said conjugate are not linked.

### Nucleic acids and vectors

As used herein, the term "polynucleotide" refers to RNA or DNA, preferably to DNA

Preferably, such a "polynucleotide" is operatively linked to a gene expression sequence, which directs the expression of the nucleic acid within a prokarotic or an eukaryotic cell, preferably within an eukaryotic cell. The "gene expression sequence" is any regulatory nucleotide sequence, such as a promoter sequence or promoter-enhancer combination, which facilitates the efficient transcription and translation of the immunocytokine nucleic acid to which it is operatively linked. The gene expression sequence may, for example, be a mammalian or viral promoter, such as a constitutive or inducible promoter.

Constitutive mammalian promoters include, but are not limited to, the promoters for the following genes: hypoxanthine phosphoribosyl transferase (HPTR), adenosine deaminase, pyruvate kinase, beta.-actin promoter, muscle creatine kinase promoter, human elongation factor promoter and other constitutive promoters. Exemplary viral promoters which function constitutively in eukaryotic cells include, for example, promoters from the simian virus (e.g., SV40), papilloma virus, adenovirus, human immunodeficiency virus (HIV), cytomegalovirus (CMV), Rous sarcoma virus (RSV), hepatitis B virus (HBV), the long terminal repeats (LTR) of Moloney leukemia virus and other retroviruses, and the thymidine kinase promoter of herpes simplex virus. Other constitutive promoters are known to those of ordinary skill in the art.

The promoters useful as gene expression sequences also include inducible promoters. Inducible promoters are expressed in the presence of an inducing agent. For example, the metallothione in promoter is induced to promote transcription and translation in the presence of certain metal ions. Others inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operationally joined nucleic acid. The gene expression sequences optionally include enhancer sequences or upstream activator sequences as desired. As used herein, the nucleic acid sequence encoding the immunocytokine of the invention and the gene expression sequence are said to be " operationally linked" when they are covalently linked in such a way as to place the expression or transcription and/or translation of the immunocytokine of the invention coding sequence under the influence or control of the gene expression sequence.

Two DNA sequences are said to be operationally linked if induction of a promoter in the 5' gene expression sequence results in the transcription of the immunocytokine of the invention and if the nature of the linkage between the two DNA sequences does not (1) result in the introduction of a frame-shift mutation, (2) interfere with the ability of the promoter region to direct the transcription of the immunocytokine of the invention, or (3) interfere with the ability of the corresponding RNA transcript to be translated into a protein. Thus, a gene expression sequence would be operationally linked to a nucleic acid sequence coding for the immunocytokine of the invention if the gene expression sequence were capable of effecting transcription of that nucleic acid sequence such that the resulting transcript is translated into the desired polypeptide.

Advantageously, said nucleic acid sequence comprises an intron, since pre-mRNA molecules has often been demonstrated to improve production yields of recombinant molecules. Any sequences of intron may be sued, and as an example, one can cite tone ones disclosed in ZAGO et al. (Biotechnol. Appl. Biochem., vol.52(Pt 3), p:191-8, 2009) and in CAMPOS-DA-PAZ et al. (Mol. Biotechnol., vol.39(2), p: 155-8, 2008).

The polynucleotide coding for the conjugate or for the immunomodulatory antibody may be delivered *in vivo* alone or in association with a vector.

In its broadest sense, a "vector" is any vehicle capable of facilitating the transfer of the nucleic acid coding for the immunocytokine of the invention to the cells. Preferably, the vector transports the nucleic acid to cells with reduced degradation relative to the extent of degradation that would result in the absence of the vector. In general, the vectors useful in the invention include, but are not limited to, plasmids, cosmids, phagmids, episomes, artificial chromosomes, viruses, other vehicles derived from viral or bacterial sources that have been manipulated by the insertion or incorporation of the immunocytokine nucleic acid sequences.

Plasmid vectors are a preferred type of vector and have been extensively described in the art and are well known to those of skill in the art. See e.g., SANBROOK et al., "Molecular Cloning: A Laboratory Manual," Second Edition, Cold Spring Harbor Laboratory Press, 1989. Not limiting examples of plasmids include pBR322, pUC18, pUC19, pRC/CMV, SV40, and pBlueScript, and other plasmids are well known to those of ordinary skill in the art. Additionally, plasmids may be custom designed using restriction enzymes and ligation reactions to remove and add specific fragments of DNA.

Preferably, the vector can include selectable markers that are active both in bacteria and in mammalian cells.

### Pharmaceutical compositions and medical uses

As used herein, the term "subject" denotes a mammal, such as a rodent, a feline, a canine or a primate, and most preferably a human.

The pharmaceutical composition of the invention may include one or more pharmaceutically acceptable carriers.

The expression "pharmaceutically acceptable" refers to molecular entities and compositions that are physiologically tolerable and do not typically produce allergic or similar undesirable reactions, such as gastric upset, dizziness and the like when administered to a human. Preferably, as used herein, the expression "pharmaceutically acceptable" means approvable by a regulatory agency of the Federal or state government or listed in the U.S. Pharmacopeia or other generally recognized pharmacopeia for use in animals, and more particularly in humans.

The term "carrier" refers to a solvent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like.

In the context of the invention, the term "treating" or "treatment", as used herein, means reversing, alleviating, inhibiting the progress of, or preventing the disorder or condition to which such term applies, or one or more symptoms of such disorder or condition. The expression "treating cancer" as used herein means the inhibition of the growth of cancer cells. Preferably such treatment also leads to the regression of tumor growth, i.e., the decrease in size of a measurable tumor. Most preferably, such treatment leads to the complete regression of the tumor.

The expression "therapeutically effective amount" refers to an amount which is sufficient to inhibit the growth of cancer cells, preferably sufficient to induce the regression of tumor growth. The administrated doses can be adapted as a function of various parameters, in particular as a function of the mode of administration used, of the relevant pathology, or alternatively of the desired duration of treatment. Naturally, the form of the pharmaceutical composition, the route of administration, the dosage and the regimen naturally depend on the condition to be treated, the severity of the illness, the age, weight, and sex of the subject, etc. The ranges of effective doses provided below are not intended to limit the invention and represent preferred dose ranges. However, the preferred dose can be tailored to the individual subject, as is understood and determinable by one of skill in the art, without undue experimentation.

In view of the marked efficiency of the combination of the invention, the skilled person can plan to use very small doses of both individual compounds for treating a subject.

As a non limiting example, the immunosuppressive receptor antagonist antibody of the invention can be can be administered by injection at a dose of 500 µg/kg or less, preferably at a dose of 100 µg/kg or less, and most preferably at a dose of 50 µg/kg or less.

Preferably, said immunosuppressive receptor antagonist antibody can be administrated by injection at a dose of at least 1 µg/kg subject, preferably of at least 5 µg/kg subject, and most preferably at a dose of at least 10 µg/kg subject.

As a non limiting example, the conjugate of the invention can be can be administered by injection at a dose of 60 µg/kg or less, preferably at a dose of 10 µg/kg or less, and most preferably at a dose of 5 µg/kg or less.

Preferably, said conjugate can be administrated by injection at a dose of at least 0.5 µg/kg subject, preferably of at least 0.7 µg/kg subject, and most preferably at a dose of at least 0.8 µg/kg subject.

As an example, the administration steps can correspond to topical, oral, intranasal, intraocular, intravenous, intramuscular, intratumoral or subcutaneous administrations and the like. Preferably, these administration steps correspond to injection. Therefore, the conjugate, the immunomodulatory antibody or fragment thereof, the polynucleotide encoding such polypeptides, or the vectors comprising such polynucleotides are associated with vehicles which are pharmaceutically acceptable for a formulation intended to be injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. Suitable pharmaceutical carriers are described in "Remington's Pharmaceutical Sciences" by E.W. Martin.

The conjugate, the immunomodulatory antibody or fragment thereof, the polynucleotide encoding such polypeptides, or the vectors comprising such polynucleotides may be solubilized in a buffer or water or incorporated in emulsions, microemulsions, hydrogels (e.g. PLGA-PEG-PLGA triblock copolymers-based hydrogels), in microspheres, in nanospheres, in microparticles, in nanoparticles (e.g. poly(lactic-co-glycolic acid) microparticles (e.g. poly lactic acid (PLA) ; poly (lactide-co-glycolic acid) (PLGA) ; polyglutamate microspheres, nanospheres, microparticles or nanoparticles), in liposomes, or other galenic formulations. In all cases, the formulation must be sterile and fluid to the extent of acceptable syringability. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose.

Dispersions can also be prepared in glycerol, liquid polyethylene glycols, and mixtures thereof and in oils. Under ordinary conditions of storage and use, these preparations contain a preservative to prevent the growth of microorganisms.

The conjugate, the immunosuppressive receptor antagonist antibody or fragment thereof, the polynucleotide encoding such polypeptides, or the vectors comprising such polynucleotides invention can be formulated into compositions in neutral or salt form. Pharmaceutically acceptable salts include the acid addition salts (formed with the free amino groups of the protein) which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or a dispersion medium containing, for example, water, ethanol, polyol (for example, glycerol, propylene glycol, and liquid polyethylene glycol, and the like), suitable mixtures thereof, and vegetables oils. The polypeptides may also be modified, by pegylation as an example, so as to increase their biodisponibility.

The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride.

Prolonged absorption of the injectable compositions can be brought about by the use in the compositions of agents delaying absorption, for example, aluminium monostearate, gelatin, polyols, and half-life enhancing covalent and non covalent formulations.

There are numerous causes of peptide instability or degradation, including hydrolysis and denaturation. Hydrophobic interaction may cause clumping of molecules together (i.e. aggregation). Stabilizers may be added to reduce or prevent such problems.

Stabilizers include cyclodextrine and derivatives thereof (see U.S. Pat. No.5,730,969). Suitable preservatives such as sucrose, mannitol, sorbitol, trehalose, dextran and glycerin can also be added to stabilize the final formulation. A stabilizer selected from ionic and non-ionic surfactants, D-glucose, D-galactose, D-xylose, D-galacturonic acid, trehalose, dextrans, hydroxyethyl starches, and mixtures thereof may be added to the formulation. Addition of alkali metal salt or magnesium chloride may stabilize a peptide. The peptide may also be stabilized by contacting it with a saccharide selected from the group consisting of dextran, chondroitin sulphuric acid, starch, glycogen, dextrin, and alginic acid salt. Other sugars that can be added include monosaccharides, disaccharides, sugar alcohols, and mixtures thereof (E.g., glucose, mannose, galactose, fructose, sucrose, maltose, lactose, mannitol, xylitol). Polyols may stabilize a peptide, and are water-miscible or water-soluble. Suitable polyols may be polyhydroxy alcohols, monosaccharides and disaccharides including mannitol, glycrol, ethylene glycol, propylene glycol, trimethyl glycol, vinyl pyrrolidone, glucose, fructose, arabinose, mannose, maltose, sucrose, and polymers thereof. Various excipients may also stabilize peptides, including serum albumin, amino acids, heparin, fatty acids and phospholipids, surfactants, metals, polyols, reducing agents, metal chelating agents, polyvinyl pyrrolidone, hydrolysed gelatin, and ammonium sulfate.

In the following, the invention is described in more detail with reference to amino acid sequences, nucleic acid sequences and examples. However, no limitation of the invention is intended by the details of the examples.

### EXAMPLES

### 1) PD1/PD-L1 cancer model

BALB/C mice were injected subcutaneously on the right flank with CT26 tumor cells (2.10⁵/mouse). On day 9, when tumours reached 20-30 mm² mice were treated with 250 µg/mouse of anti-PD1 (BIOXCELL, clone RPM1-14, #BE0146) alone or isotype control (rat IgG2a; BIOXCELL, clone 2A3, #BE0089). Anti-PD1 (α-PD1) or isotype control (2A3) was injected at day 9, 12 and 15. From 13 to 37, mice were treated twice a week with 2 µg of RLI CHO (1004-14p) or PBS in control groups. Tumors were measured three times per weeks with a calliper and tumor area was calculated as follow: length x width. Mice were sacrificed when tumour size reached 300 mm² or were ulcerated.

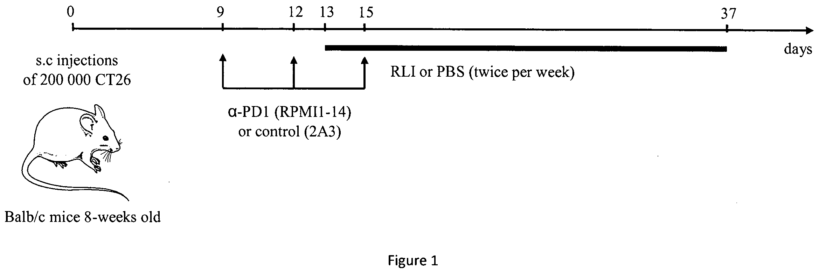

The protocol is presented in figure 1. CT26 tumor-bearing mice received three 3 doses (250 µg/dose) of anti-PD1 or isotype control from day 9 to 15. RLI treatment (2µg/dose) was injected from day 13 to day 37 twice per week.

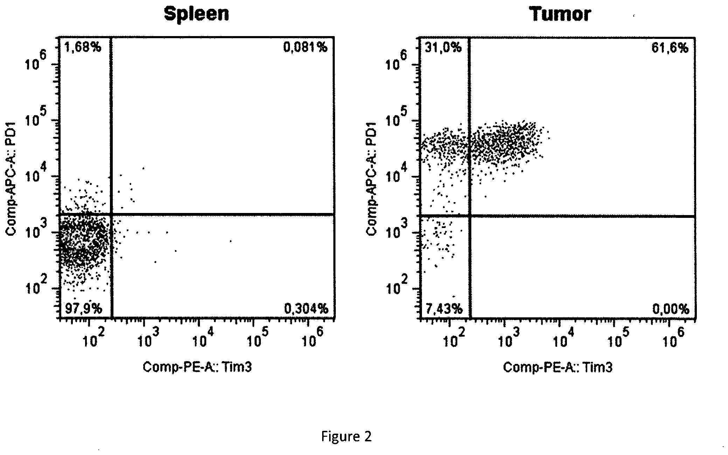

The CT26 tumor model expressed PD-L1 (ligand for PD1; (DURAISWAMY et al., Cancer Res., Jun 5, 2013) and is infiltrated by T cells highly expressing the PD1 receptor. The figure 2 shows the high frequency of tumor infiltrating CD8+ T cells co-express of PD-1 and Tim-3 in the CT26 model. Dissociated spleen and tumor from CT26 tumor-bearing mouse were stained and analyzed by flow cytometry. Co-expression of Tim-3 and PD-1 on CD3+ CD8+ T cells is shown in spleen (left panel) and in the tumor (right panel).

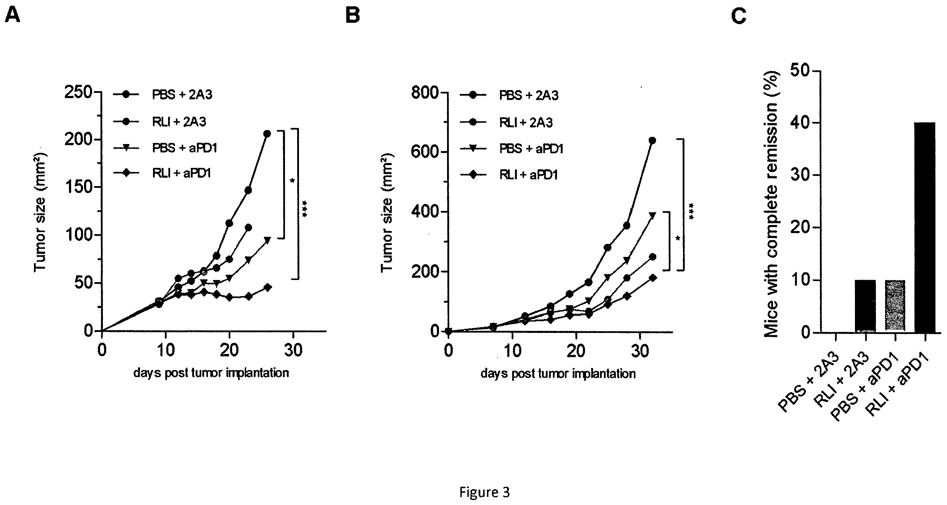

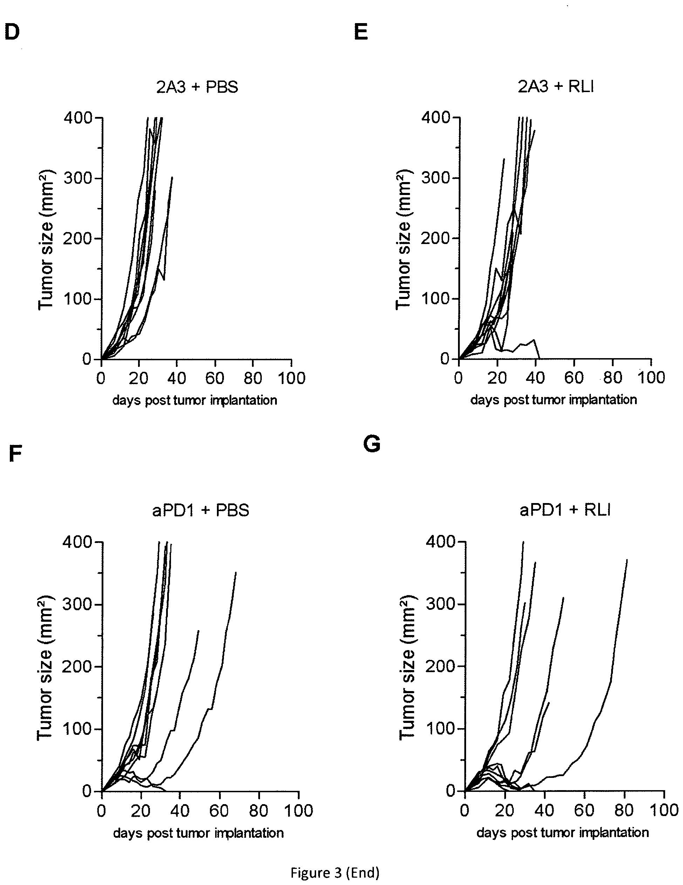

The figure 3 shows the tumor size evolution in CT26 tumor-bearing mice treated with anti-PD1 (aPD1) or control (2A3), RLI or control (PBS) or combining both treatments (aPD1 + RLI) according to previously described protocol. (A) Tumor growth in each group (n=5 mice/group) (B) As in A. but from a second experimentation. C. Percentage of mice with complete tumor regression in each group. (D-G). Tumor growth in each mouse (pooled of experimentation 1 and 2, n=10) for the control treated group (D); for the RLI standalone treated group (F); for the aPD1 standalone treated group (F) and for the aPD1 + RLI treated group (F). Statistical tests were determined using 2-way ANOVA. * p<0.05; *** p<0,001.

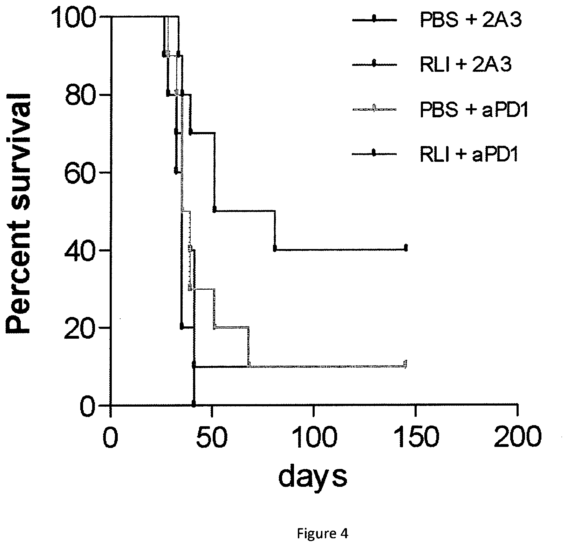

The figure 4 shows that the anti-PD1 / RLI combination treatment increases overall survival in mice. A. Overall survival in each treated group (n=10 per group).

Thus, the results show that anti-PD1+RLI combination treatment enhances antitumor effect compare to standalone groups. In particular, RLI/aPD1 treatment increases the number of mice with complete tumor regression and overall survival (Figure 3C and 4). Surprisingly, no tumour free mice treated with a combination of RLI and anti-PD1 rechallenged at day 145 after total remission with CT26 tumors, showed any tumor growth suggesting the persistence of a protective anti-tumour immunity (data not shown). These results were confirmed in another series of experiments establishing that i) at stand alone, RLI had no effect on the tumor growth when treatment started from D10 after tumor inoculation, ii) At that time, anti-PD1 enhanced survival but rarely induced complete regression (5.9%). The combination of anti-PD1 and RLI increased survival and complete remissions (30,4%), which were long-lasting remissions. This combination induced an effective antitumor memory immune response since the mice were still tumor free 145 days after the total regression and re-challenge of cured mice never showed tumor recovery (data not shown).

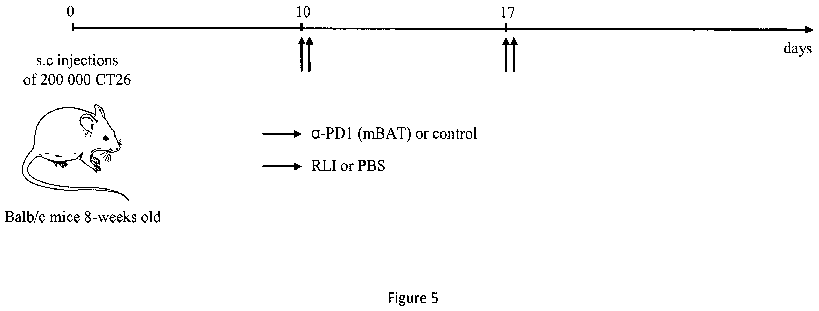

BALB/C mice were injected subcutaneously on the right flank with CT26 tumor cells (2.10⁵/mouse). Mice received i.p. low dose anti-PD1 (clone mBAT, 12 µg/mouse) or control, 2µg RLI CHO (1004-15p) or control or combination of both treatments at day 10. One week later (day 17) mice receive a second injection of anti-PD1, RLI or controls. Tumors were measured every two days with a calliper and tumour area was calculated as follow: length x width. Mice were scarified when tumour size reached 300 mm² or were ulcerated. The protocol is presented in figure 5.

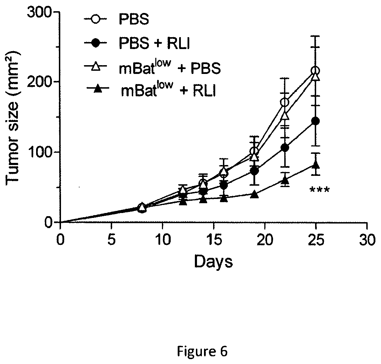

The figure 6 shows the CT26 tumor growth in CT26 tumor-bearing mice treated with two i.p injections of anti-PD1 at low in combination with RLI. Statistical tests were determined using 2-way ANOVA. *** *p*<0,001.

The results show that a low dose of anti-PD1 or RLI alone is unable to support the inhibition of primary subcutaneous tumor growth. However, combining low dose anti-PD1 with RLI allowed tumor growth inhibition. This experiment confirmed that RLI could synergize with anti-PD1 strategies to combat tumor growth even with a suboptimal dose of anti-PD1 treatment.

Finally, it seems that this low-doses combination results in a marked synergy resulting in a strong tumor growth inhibition.

### 2) Metastatic melanoma

B16F10 tumors are implanted by injection of 3 × 10⁴ B16F10 cells in the flank of C57BL/6 mice, i.d. at day 0. Mice are treated i.p. with anti-CTLA-4 (clone: UC10-4F10-11 100 or clone: 9D9) or with the anti-PD-1 (clone RPM1-14, #BE0146) or with the anti-PD-L1 mAb (clone: 10F.9G2) in combination or not with RLI CHO (2 µg/mouse) i.p. injected from day 7 to 25 twice a week with 2 µg of RLI CHO. Dosing of anti-CTLA-4 or anti-PD-1 or anti-PD-L1 antibody per injection is 200 µg on day 3 plus 100 µg on days 6 and 9. Control groups received a corresponding dose of antibody isotype. Tumor size and incidence were monitored over time by physical examination.

### 3) Advanced lung cancer

The tumor cell line, TC-1 was derived from primary lung epithelial cells of C57BL/6 mice and is human papillomavirus 16 (HPV-16) E6/E7 and c-Ha-Ras cotransformed. 10⁵ TC-1 lung cancer cells are injected s.c. into the upper dermis in the back of C57BL/6 mice. Treatment is initiated on day 10 after tumor inoculation which corresponds to when tumors become clearly visible and palpable at a size of ≈ 15-20 mm2. The anti-CTLA-4 mAb (100 µg/mouse, clone: UC10-4F10-11 100 or clone: 9D9) or the anti-PD-1 (250 µg/mouse, clone RPM1-14, #BE0146) or the anti-PD-L1 mAb (100 µg/mouse, clone: 10F.9G2) is given at three single time points (day 10, 14, 17), and RLI (2 µg/mouse) is given at day 10, 13 and 18. Treatment with single agents versus doublet combinations of each antibody with RLI is compared, using tumor growth measured twice per week as the endpoint. Control groups received a corresponding dose of antibody isotype.

### 4) Advanced Bladder Cancer

The MB49 tumour cell line originates from a carcinogen-induced tumour of bladder epithelial origin from C57BL/6 male mice. 10⁶ MB49 bladder cancer cells are injected s.c. into the upper dermis in the back of C57BL/6 mice. Treatment is initiated on day 6 after tumor inoculation which corresponds to when tumors become clearly visible and palpable at a size of ≈ 15 mm2. The anti-CTLA-4 mAb (100 µg/mouse, clone: UC10-4F10-11 100 or clone: 9D9) or the anti-PD-1 (100 µg/mouse, clone RPM1-14, #BE0146) or the anti-PD-L1 mAb (100 µg/mouse, clone: 10F.9G2) is given at four single time points (day 6, 9, 12), and RLI (2 µg/mouse) is given at day 7, 8, 10, 11, 13, 14, and 16, 17. Treatment with single agents versus doublet combinations of each antibody with RLI is compared. Control groups received a corresponding dose of antibody isotype. Tumors were measured three times per weeks with a calliper and tumor area was calculated as follow: length x width. Mice were sacrificed when tumour size reached 300 mm² or were ulcerated.

### 5) Breast Cancer

BALB/c mice are inoculated with 5 × 10⁴ 4T1 breast cancer cells in the mammary gland on day 0. Treatment is initiated on day 10 after tumor inoculation which corresponds to when tumors become clearly visible and palpable at a size of ≈ 15-20 mm2. The anti-CTLA-4 mAb (100 µg/mouse, clone: UC10-4F10-11 100 or clone: 9D9) or the anti-PD-1 (250 µg/mouse, clone RPM1-14, #BE0146) or the anti-PD-L1 mAb (200 µg/mouse, clone: 10F.9G2) is given at four single time points (day 10, 13, 16), and RLI (2 µg/mouse) is given at day 10, 11, 13, 14, 16, 17, and 19, 20. Treatment with single agents versus doublet combinations of each antibody with RLI is compared. Control groups received a corresponding dose of antibody isotype. Tumors are measured three times per weeks with a calliper and tumor area is calculated as follow: length x width. Mice are sacrificed at day 27. Lung metastatic nodules are counted under a binocular microscope.

### 6) Ovary cancer

ID8-VEGF ovarian carcinoma cell line was developed previously from a mouse ovarian epithelial papillary serous adenocarcinoma cell line. C57BL/6 mice are implanted subcutaneously on the right flank with either 5 × 10⁶ ID8 tumor cells. Treatment is initiated on day 10 after tumor inoculation which corresponds to when tumors become clearly visible and palpable at a size of ≈ 15-20 mm2. The anti-CTLA-4 mAb (100 µg/mouse, clone: UC10-4F10-11 100 or clone: 9D9) or the anti-PD-1 (250 µg/mouse, clone RPM1-14, #BE0146) or the anti-PD-L1 mAb (200 µg/mouse, clone: 10F.9G2) is given at four single time points (day 10, 13, 16), and RLI (2 µg/mouse) is given at day 10, 11, 13, 14, 16, 17, and 19, 20. Treatment with single agents versus doublet combinations of each antibody with RLI is compared, using tumor growth measured twice per week as the endpoint. Control groups received a corresponding dose of antibody isotype. Tumors are measured three times per weeks with a calliper and tumor area is calculated as follow: length x width. Mice are sacrificed when tumour size reached 300 mm² or are ulcerated.

### 7) Prostate cancer: RM-1

C57BL/6 female mice are inoculated s.c. with 2 × 10⁵ RM-1 murine prostate cancer cells. Treatment is initiated on day 3 after tumor inoculation which corresponds to when tumors become clearly visible and palpable at a size of ≈ 15-20 mm2. The anti-CTLA-4 mAb (100 µg/mouse, clone: UC10-4F10-11 100 or clone: 9D9) or the anti-PD-1 (250 µg/mouse, clone RPM1-14, #BE0146) or the anti-PD-L1 mAb (200 µg/mouse, clone: 10F.9G2) is given at three single time points (day 3, 6, 9), and RLI (2 µg/mouse) is given at day 6, 7, 9, 10, 12, 13, 15, 16 and 18, 19. Treatment with single agents versus doublet combinations of each antibody with RLI is compared, using tumor growth measured twice per week as the endpoint. Control groups received a corresponding dose of antibody isotype. Tumors are measured three times per weeks with a calliper and tumor area is calculated as follow: length x width. Mice were sacrificed when tumour size reached 300 mm² or were ulcerated.

## Claims

1. A combined pharmaceutical composition comprising:
a) a conjugate comprising (i) a polypeptide comprising the amino acid sequence of interleukin 15 or derivatives thereof having at least 10% of the activity of human IL-15 on the proliferation induction of kit225 cell line, and (ii) a polypeptide comprising the amino acid sequence of the sushi domain of IL-15Rα or derivatives thereof having at least 10% of the binding activity of the sushi domain of human IL-15Rα to human IL-15, a polynucleotide coding therefore, or a vector comprising such a polynucleotide; and
b) an antibody antagonizing an immune pathway implicated in the inhibition of T cell activation, or a fragment thereof capable of reacting with the same antigen than its antibody counterpart, a polynucleotide coding therefore, or a vector comprising such a polynucleotide,
wherein said antibody is a PD-1/PD-L1/PD-L2 antagonist,
wherein said antibody is antagonizing either by binding the PD-1 receptor or by binding the PD-L1 or PD-L2 ligand;
wherein said conjugate and the antibody or fragment thereof are not linked.

2. A pharmaceutical composition for use in treating cancer in a subject comprising
(A) a conjugate comprising (i) a polypeptide comprising the amino acid sequence of interleukin 15 or derivatives thereof having at least 10% of the activity of human IL-15 on the proliferation induction of kit225 cell line, and (ii) a polypeptide comprising the amino acid sequence of the sushi domain of IL-15Rα or derivatives thereof having at least 10% of the binding activity of the sushi domain of human IL-15Rα to human IL-15, a polynucleotide coding therefore, or a vector comprising such a polynucleotide,
wherein the use comprises simultaneously, separately, or sequentially administering (a) said pharmaceutical composition and (b) an antibody antagonizing an immune pathway implicated in the inhibition of T cell activation, or a fragment thereof which is capable of reacting with the same antigen than its antibody counterpart, a polynucleotide coding therefore, or a vector comprising such a polynucleotide, or
(B) an antibody antagonizing an immune pathway implicated in the inhibition of T cell activation, or a fragment thereof which is capable of reacting with the same antigen than its antibody counterpart, a polynucleotide coding therefore, or a vector comprising such a polynucleotide,
wherein the use comprises simultaneously, separately, or sequentially administering (a) said pharmaceutical composition and (b) a conjugate comprising (i) a polypeptide comprising the amino acid sequence of interleukin 15 or derivatives thereof having at least 10% of the activity of human IL-15 on the proliferation induction of kit225 cell line, and (ii) a polypeptide comprising the amino acid sequence of the sushi domain of IL-15Rα or derivatives thereof having at least 10% of the binding activity of the sushi domain of human IL-15Rα to human IL-15, a polynucleotide coding therefore, or a vector comprising such a polynucleotide, and
wherein said antibody is a PD-1/PD-L1/PD-L2 antagonist,
wherein said antibody is antagonizing either by binding the PD-1 receptor or by binding the PD-L1 or PD-L2 ligand.

3. The pharmaceutical composition for use in treating cancer of claim 2 or the combined pharmaceutical composition of claim 1, adapted for simultaneous, separate, or sequential administration for use in treating cancer in a subject.

4. The pharmaceutical composition for use in treating cancer or the combined pharmaceutical composition of any one of claims 1 to 3, wherein:
a) the conjugate is administrated by injection at a dose of 60 µg/kg or less, preferably at a dose of 10 µg/kg or less and most preferably at a dose of 5 µg/kg or less; and
b) the antibody antagonizing an immune pathway implicated in the inhibition of T cell activation, or a fragment thereof is administrated by injection at a dose of 500 µg/kg or less, preferably at a dose of 100 µg/kg or less and most preferably at a dose of 50 µg/kg or less.

5. The pharmaceutical composition for use in treating cancer or the combined pharmaceutical composition of any one of claims 1 to 4, wherein said antibody is a PD-1/PD-L1 antagonist.

6. The pharmaceutical composition for use in treating cancer or the combined pharmaceutical composition of any one of claims 1 to 5, wherein said interleukin 15 derivative has at least 25% of the activity of human interleukin-15 on the proliferation induction of kit225 cell line, more preferably at least 50%, or
has an amino acid sequence having a percentage of identity of at least 92.5% with an amino acid sequence selected in the group consisting of SEQ ID n°: 1, SEQ ID n°: 2 and SEQ ID n°: 3, preferably of at least 96 %, and more preferably of at least 98.5% or of at least 99%.

7. The pharmaceutical composition or the combined pharmaceutical composition of any one of the claims 1 to 6, wherein said derivative of the sushi domain of IL-15Rα has at least 25%, more preferably at least 50% of the binding activity of the sushi domain of human IL-15Rα, or
has an amino acid sequence having a percentage of identity of at least 92% with an amino acid sequence selected in the group consisting of SEQ ID n°: 4, SEQ ID n°: 5, SEQ ID n°: 6, SEQ ID n°: 7, SEQ ID n°: 8, and SEQ ID n°: 9, preferably of at least 96 %, and more preferably of at least 98%.

8. The pharmaceutical composition for use in treating cancer or the combined pharmaceutical composition of any one of claims 1 to 7, wherein said antibody is selected among PD-1/PD-L1 and PD-1/PD-L2 antagonists, preferably nivolumab, Merck 3745, CT-01 1, lambrolizumab, AMP514, MDX- 1 105 or YW243.55.S70.

9. The pharmaceutical composition for use in treating cancer or the combined pharmaceutical composition of any one claims 1 to 8, wherein the polypeptides (i) and (ii) of the conjugate are covalently linked in a fusion protein.

10. The pharmaceutical composition for use in treating cancer or the combined pharmaceutical composition of any one of claims 1 to 9, wherein said interleukin 15 has the amino acid sequence SEQ ID n°: 1.

11. The pharmaceutical composition for use in treating cancer or the combined pharmaceutical composition of any one of claims 1 to 10, wherein sushi domain of IL-15Rα has the amino acid sequence SEQ ID n°: 4.

12. The pharmaceutical composition for use in treating cancer or the combined pharmaceutical composition of any one of claims 1 to 11, wherein the conjugate has the sequence SEQ ID n°: 16 or SEQ ID n°: 17.

## Patentansprüche

1. Eine kombinierte pharmazeutische Zusammensetzung, umfassend:
a) ein Konjugat, umfassend (i) ein Polypeptid, umfassend die Aminosäuresequenz von Interleukin 15 oder Derivaten davon mit mindestens 10% der Aktivität von humanem IL-15 bei der Proliferationsinduktion der Kit225-Zellinie, und (ii) ein Polypeptid, umfassend die Aminosäuresequenz der Sushi-Domäne von IL-15Rα oder Derivaten davon mit mindestens 10% der Bindungsaktivität der Sushi-Domäne von humanem IL-15Rα an humanes IL-15, ein dafür kodierendes Polynukleotid oder einen Vektor, umfassend ein solches Polynukleotid; und
b) einen Antikörper, der einen Immunpfad antagonisiert, der an der Hemmung der T-Zell-Aktivierung beteiligt ist, oder ein Fragment davon, das in der Lage ist, mit dem gleichen Antigen wie sein Antikörpergegenstück zu reagieren, einem dafür kodierenden Polynukleotid oder einem Vektor, der ein solches Polynukleotid umfasst,
wobei besagter Antikörper ein PD-1/PD-L1/PD-L2-Antagonist ist,
wobei besagter Antikörper entweder durch Bindung des PD-1-Rezeptors oder durch Bindung des PD-L1- oder PD-L2-Liganden antagonisierend ist;
wobei das Konjugat und der Antikörper oder das Fragment davon nicht verbunden sind.

2. Eine pharmazeutische Zusammensetzung zur Verwendung bei der Behandlung von Krebs bei einem Individuum, umfassend
(A) ein Konjugat, umfassend (i) ein Polypeptid, umfassend die Aminosäuresequenz von Interleukin 15 oder Derivaten davon mit mindestens 10% der Aktivität von humanem IL-15 bei der Proliferationsinduktion der Kit225-Zellinie, und (ii) ein Polypeptid, umfassend die Aminosäuresequenz der Sushi-Domäne von IL-15Rα oder Derivaten davon mit mindestens 10% der Bindungsaktivität der Sushi-Domäne von humanem IL-15Rα an humanes IL-15, ein dafür kodierendes Polynukleotid oder einen Vektor, umfassend ein solches Polynukleotid,
wobei die Verwendung das gleichzeitige, getrennte oder aufeinanderfolgende Verabreichen (a) besagter pharmazeutischer Zusammensetzung und (b) eines Antikörpers umfasst, der einen Immunpfad antagonisiert, der an der Hemmung der T-Zell-Aktivierung beteiligt ist, oder eines Fragments davon, das in der Lage ist, mit dem gleichen Antigen wie sein Antikörpergegenstück zu reagieren, einem dafür kodierenden Polynukleotid oder einem Vektor, der ein solches Polynukleotid umfasst, oder
(B) einen Antikörper, der einen Immunpfad antagonisiert, der an der Hemmung der T-Zell-Aktivierung beteiligt ist, oder ein Fragment davon, das in der Lage ist, mit dem gleichen Antigen wie sein Antikörpergegenstück zu reagieren, einem dafür kodierenden Polynukleotid oder einem Vektor, der ein solches Polynukleotid umfasst,
wobei die Verwendung das gleichzeitige, getrennte oder aufeinanderfolgende Verabreichen (a) besagter pharmazeutischer Zusammensetzung und (b) eines Konjugats umfasst, das (i) ein Polypeptid umfasst, das die Aminosäuresequenz von Interleukin 15 oder Derivaten davon mit mindestens 10% der Aktivität von menschlichem IL-15 bei der Proliferationsinduktion der Kit225-Zellinie umfasst, und (ii) ein Polypeptid, umfassend die Aminosäuresequenz der Sushi-Domäne von IL-15Rα oder Derivaten davon mit mindestens 10% der Bindungsaktivität der Sushi-Domäne von humanem IL-15Rα an humanes IL-15, ein dafür kodierendes Polynukleotid oder einen Vektor, der ein solches Polynukleotid umfasst, und
wobei der Antikörper ein PD-1/PD-L1 /PD-L2-Antagonist ist,
wobei der Antikörper entweder durch Bindung des PD-1-Rezeptors oder durch Bindung des PD-L1- oder PD-L2-Liganden antagonisierend ist.

3. Die pharmazeutische Zusammensetzung zur Verwendung bei der Behandlung von Krebs gemäß Anspruch 2 oder die kombinierte pharmazeutische Zusammensetzung gemäß Anspruch 1, angepasst für die gleichzeitige, getrennte oder aufeinanderfolgende Verabreichung zur Verwendung bei der Behandlung von Krebs bei einem Individuum.

4. Die pharmazeutische Zusammensetzung zur Verwendung bei der Behandlung von Krebs oder die kombinierte pharmazeutische Zusammensetzung gemäß einem der Ansprüche 1 bis 3, wobei:
a) das Konjugat durch Injektion in einer Dosis von 60 µg/kg oder weniger, vorzugsweise in einer Dosis von 10 µg/kg oder weniger und am bevorzugtesten in einer Dosis von 5 µg/kg oder weniger verabreicht wird; und
b) der Antikörper, der einen Immunpfad antagonisiert, der an der Hemmung der T-Zell-Aktivierung beteiligt ist, oder ein Fragment davon durch Injektion in einer Dosis von 500 µg/kg oder weniger, vorzugsweise in einer Dosis von 100 µg/kg oder weniger und am bevorzugtesten in einer Dosis von 50 µg/kg oder weniger, verabreicht wird.

5. Die pharmazeutische Zusammensetzung zur Verwendung bei der Behandlung von Krebs oder die kombinierte pharmazeutische Zusammensetzung gemäß einem der Ansprüche 1 bis 4, wobei der Antikörper ein PD-1/PD-L1-Antagonist ist.

6. Die pharmazeutische Zusammensetzung zur Verwendung bei der Behandlung von Krebs oder die kombinierte pharmazeutische Zusammensetzung gemäß einem der Ansprüche 1 bis 5, wobei das Interleukin-15-Derivat mindestens 25% der Aktivität von humanem Interleukin-15 bei der Proliferationsinduktion der Kit225-Zellinie, bevorzugter mindestens 50%, aufweist, oder
eine Aminosäuresequenz mit einem Identitätsprozentsatz von mindestens 92,5% mit einer Aminosäuresequenz aufweist, die aus der Gruppe ausgewählt ist, die aus SEQ ID n°: 1, SEQ ID n°: 2 und SEQ ID n°: 3 besteht, vorzugsweise von mindestens 96 %, und bevorzugter von mindestens 98,5 % oder von mindestens 99 %.

7. Die pharmazeutische Zusammensetzung oder die kombinierte pharmazeutische Zusammensetzung gemäß einem der Ansprüche 1 bis 6, wobei das Derivat der Sushi-Domäne von IL-15Rα mindestens 25%, vorzugsweise mindestens 50% der Bindungsaktivität der Sushi-Domäne von humanem IL-15Rα aufweist, oder
eine Aminosäuresequenz mit einem Identitätsprozentsatz von mindestens 92% mit einer Aminosäuresequenz aufweist, die aus der Gruppe ausgewählt ist, bestehend aus SEQ ID n°: 4, SEQ ID n°: 5, SEQ ID n°: 6, SEQ ID n°: 7, SEQ ID n°: 8 und SEQ ID n°: 9, vorzugsweise von mindestens 96 % und bevorzugter von mindestens 98 %.

8. Die pharmazeutische Zusammensetzung zur Verwendung bei der Behandlung von Krebs oder die kombinierte pharmazeutische Zusammensetzung gemäß einem der Ansprüche 1 bis 7, wobei besagter Antikörper ausgewählt ist aus PD-1/PD-L1- und PD-1/PD-L2-Antagonisten, vorzugsweise Nivolumab, Merck 3745, CT-01 1, Lambrolizumab, AMP514, MDX- 1 105 oder YW243.55.S70.

9. Die pharmazeutische Zusammensetzung zur Verwendung bei der Behandlung von Krebs oder die kombinierte pharmazeutische Zusammensetzung gemäß einem der Ansprüche 1 bis 8, wobei die Polypeptide (i) und (ii) des Konjugats in einem Fusionsprotein kovalent verknüpft sind.

10. Die pharmazeutische Zusammensetzung zur Verwendung bei der Behandlung von Krebs oder die kombinierte pharmazeutische Zusammensetzung gemäß einem der Ansprüche 1 bis 9, wobei das Interleukin 15 die Aminosäuresequenz SEQ ID n°: 1 aufweist.

11. Die pharmazeutische Zusammensetzung zur Verwendung bei der Behandlung von Krebs oder die kombinierte pharmazeutische Zusammensetzung gemäß einem der Ansprüche 1 bis 10, wobei die Sushi-Domäne von IL-15Rα die Aminosäuresequenz SEQ ID n°: 4 aufweist.

12. Die pharmazeutische Zusammensetzung zur Verwendung bei der Behandlung von Krebs oder die kombinierte pharmazeutische Zusammensetzung gemäß einem der Ansprüche 1 bis 11, wobei das Konjugat die Sequenz SEQ ID n°: 16 oder SEQ ID n°: 17 aufweist.

## Revendications

1. Composition pharmaceutique combinée comprenant :
a) un conjugué comprenant (i) un polypeptide qui comprend la séquence d'acides aminés de l'interleukine 15 ou de ses dérivés ayant au moins 10 % de l'activité de l'IL-15 humaine sur l'induction de la prolifération de la lignée cellulaire kit225, et (ii) un polypeptide comprenant la séquence d'acides aminés du domaine sushi de l'IL-15Rα ou de ses dérivés ayant au moins 10 % de l'activité de liaison du domaine sushi de l'IL-15Rα humain à l'IL-15 humaine, un polynucléotide codant pour celle-ci, ou un vecteur comprenant ce polynucléotide ; et
b) un anticorps antagonisant une voie immunitaire impliquée dans l'activation de lymphocytes T, ou un de ses fragments capable de réagir avec le même antigène que sa contrepartie d'anticorps, un polynucléotide codant pour celui-ci, ou un vecteur comprenant ce polynucléotide,
dans laquelle ledit anticorps est un antagoniste de PD-1/PD-L1/PD-L2,
dans laquelle ledit anticorps antagonise par la liaison au récepteur de PD1 ou par la liaison du ligand PD-L1 ou PD-L2 ;
dans laquelle ledit conjugué et l'anticorps ou un de ses fragments ne sont pas liés.

2. Composition pharmaceutique destinée à être utilisée dans le traitement du cancer chez un sujet comprenant :
(A) un conjugué comprenant (i) un polypeptide qui comprend la séquence d'acides aminés de l'interleukine 15 ou de ses dérivés ayant au moins 10 % de l'activité de l'IL-15 humaine sur l'induction de la prolifération de la lignée cellulaire kit225, et (ii) un polypeptide comprenant la séquence d'acides aminés du domaine sushi de l'IL-15Rα ou de ses dérivés ayant au moins 10 % de l'activité de liaison du domaine sushi de l'IL-15Rα humain à l'IL-15 humaine, un polynucléotide codant pour celui-ci, ou un vecteur comprenant ce polynucléotide,
dans laquelle l'utilisation comprend simultanément, séparément ou séquentiellement l'administration (a) de ladite composition pharmaceutique et (b) d'un anticorps antagonisant une voie immunitaire impliquée dans l'inhibition de l'activation de lymphocytes T, ou un de ses fragments qui est capable de réagir avec le même antigène que sa contrepartie d'anticorps, un polynucléotide codant pour celui-ci, ou un vecteur comprenant ce polynucléotide, ou
(B) un anticorps antagonisant une voie immunitaire impliquée dans l'activation de lymphocytes T, ou un de ses fragments qui est capable de réagir avec le même antigène que sa contrepartie d'anticorps, un polynucléotide codant pour celui-ci, ou un vecteur comprenant ce polynucléotide,
dans laquelle l'utilisation comprend simultanément, séparément ou séquentiellement l'administration (a) de ladite composition pharmaceutique et (b) d'un conjugué comprenant (i) un polypeptide qui comprend la séquence d'acides aminés de l'interleukine 15 ou de ses dérivés ayant au moins 10 % de l'activité de l'IL-15 humaine sur l'induction de la prolifération de la lignée cellulaire kit225, et (ii) un polypeptide comprenant la séquence d'acides aminés du domaine sushi de l'IL-15Rα ou ses dérivés ayant au moins 10 % de l'activité de liaison du domaine sushi de l'IL-15Rα humain à l'IL-15 humaine, un polynucléotide codant pour celui-ci, ou un vecteur comprenant ce polynucléotide, et
dans laquelle ledit anticorps est un antagoniste de PD-1/PD-L1/PD-L2,
dans laquelle ledit anticorps antagonise par liaison au récepteur de PD1 ou par liaison du ligand PD-L1 ou PD-L2.

3. Composition pharmaceutique destinée à être utilisée dans le traitement du cancer selon la revendication 2 ou composition pharmaceutique combinée selon la revendication 1, adaptée pour l'administration simultanée, séparée ou séquentielle destinée à être utilisée dans le traitement du cancer chez un sujet.

4. Composition pharmaceutique destinée à être utilisée dans le traitement du cancer ou composition pharmaceutique combinée selon l'une quelconque des revendications 1 à 3, dans laquelle :
a) le conjugué est administré par une dose de 60 µg/kg ou moins, de préférence à une dose de 10 µg/kg ou moins et de préférence entre toutes à une dose de 5 µg/kg ou moins ; et
b) l'anticorps antagonisant une voie immunitaire impliquée dans l'inhibition de l'activation de lymphocytes T ou un de ses fragments est administré par injection à une dose de 500 µg/kg ou moins, de préférence à une dose de 100 µg/kg ou moins et de préférence entre toutes à une dose de 50 µg/kg ou moins.

5. Composition pharmaceutique destinée à être utilisée dans le traitement du cancer ou composition pharmaceutique combinée selon l'une quelconque des revendications 1 à 4, dans laquelle ledit anticorps est un antagoniste de PD-1/PD-L1.

6. Composition pharmaceutique destinée à être utilisée dans le traitement du cancer ou composition pharmaceutique combinée selon l'une quelconque des revendications 1 à 5, dans laquelle ledit dérivé d'interleukine 15 a au moins 25 % de l'activité de l'interleukine 15 humaine sur l'induction de la prolifération de la lignée cellulaire kit225, davantage de préférence au moins 50 %, ou
a une séquence d'acides aminés ayant un pourcentage d'identité d'au moins 92,5 % avec une séquence d'acides aminés choisie dans le groupe constitué par SEQ ID n° 1, SEQ ID n° 2 et SEQ ID n° 3, de préférence au moins 96 %, et davantage de préférence d'au moins 98,5 % ou d'au moins 99 %.

7. Composition pharmaceutique ou composition pharmaceutique combinée selon l'une quelconque des revendications 1 à 6, dans laquelle ledit dérivé du domaine sushi de l'IL-15Rα a au moins 25 %, davantage de préférence au moins 50 % de l'activité de liaison du domaine sushi de l'IL-15Rα humain, ou
a une séquence d'acides aminés ayant un pourcentage d'identité d'au moins 92 % avec une séquence d'acides aminés choisie dans le groupe constitué par SEQ ID n° 4, SEQ ID n° 5, SEQ ID n° 6, SEQ ID n° 7, SEQ ID n° 8 et SEQ ID n° 9, de préférence d'au moins 96 % et davantage de préférence d'au moins 98 %.

8. Composition pharmaceutique destinée à être utilisée dans le traitement du cancer ou composition pharmaceutique combinée selon l'une quelconque des revendications 1 à 7, dans laquelle ledit anticorps est choisi parmi les antagonistes de PD1/PD-L1 et PD-1/PD-L2, de préférence le nivolamab, Merck 3745, CT-01 1, le lambrolizumab, AMP514, MDX-1 105 ou YW243.55.S70.

9. Composition pharmaceutique destinée à être utilisée dans le traitement du cancer ou composition pharmaceutique combinée selon l'une quelconque des revendications 1 à 8, dans laquelle les polypeptides (i) et (ii) du conjugué sont liés de manière covalente dans une protéine de fusion.

10. Composition pharmaceutique destinée à être utilisée dans le traitement du cancer ou composition pharmaceutique combinée selon l'une quelconque des revendications 1 à 9, dans laquelle ladite interleukine 15 a la séquence d'acides aminés de SEQ ID n° 1.

11. Composition pharmaceutique destinée à être utilisée dans le traitement du cancer ou composition pharmaceutique combinée selon l'une quelconque des revendications 1 à 10, dans laquelle le domaine sushi de l'IL-15Rα a la séquence d'acides aminés de SEQ ID n° 4.

12. Composition pharmaceutique destinée à être utilisée dans le traitement du cancer ou composition pharmaceutique combinée selon l'une quelconque des revendications 1 à 11, dans laquelle le conjugué a la séquence de SEQ ID n° 16 ou de SEQ ID n° 17.
